(19) Europäisches Patentamt
European Patent Office
Office européen des brevets

(11) EP 1 564 288 B1

(12) **EUROPEAN PATENT SPECIFICATION**

(45) Date of publication and mention of the grant of the patent:
**07.04.2010 Bulletin 2010/14**

(21) Application number: **05005854.4**

(22) Date of filing: **02.02.1998**

(51) Int Cl.:
*C12N 15/09* (2006.01) *C12N 15/54* (2006.01)
*C12N 9/12* (2006.01) *G06F 17/00* (2006.01)
*G06F 19/00* (2006.01) *G06F 15/00* (2006.01)

(54) **Function-modified thermophilic DNA polymerase**

Thermophile DNA Polymerase mit modifizierter Funktion

ADN polymérase thermophile a fonction modifiée

(84) Designated Contracting States:
**CH DE FR GB LI NL**

(30) Priority: **31.01.1997 JP 1924897**
**31.01.1997 JP 1924997**
**02.12.1997 JP 33210097**
**30.01.1998 JP 1869998**

(43) Date of publication of application:
**17.08.2005 Bulletin 2005/33**

(62) Document number(s) of the earlier application(s) in accordance with Art. 76 EPC:
**98901095.4 / 1 013 759**

(73) Proprietor: **Japan Science and Technology Agency**
**Kawaguchi-shi,**
**Saitama 332-0012 (JP)**

(72) Inventors:
- **Doi, Hirofumi**
  **Chiba 273-0865 (JP)**
- **Kanai, Akio**
  **Tsuruoka-shi,**
  **Yamagata (JP)**
- **Hiraki, Hideaki**
  **Tokyo 113-0022 (JP)**

(74) Representative: **Albutt, Jodie et al**
**Dehns**
**St Bride's House**
**10 Salisbury Square**
**London**
**EC4Y 8JD (GB)**

(56) References cited:
- **DATABASE GENESEQ AC No: R96733 15 October 1996 (1996-10-15), MATHUR EJ: "Wild-type P. furiosus DNA polymerase I" XP002164692**
- **ZHANG B ET AL: "FROM FOLD PREDICTIONS TO FUNCTION PREDICTIONS: AUTOMATION OF FUNCTIONAL SITE CONSERVATION ANALYSIS FOR FUNCTIONAL GENOME PREDICTIONS" PROTEIN SCIENCE,CAMBRIDGE UNIVERSITY PRESS, CAMBRIDGE,GB, vol. 8, March 1999 (1999-03), pages 1104-1115, XP002923809 ISSN: 0961-8368**

EP 1 564 288 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present invention relates to a thermophilic DNA polymerase prepared by artificially modifying the amino acid sequence of Pfu DNA polymerase. A method for predicting a functional site of a protein, a system for predicting the function thereof, and a method for modifying the function of a protein have been developed and a function-modified protein is disclosed herein. The prediction of a functional site of a protein with no information on function obtained by genome analysis or cDNA analysis, and prediction of a novel function or a novel functional site of a protein with a known function can be achieved. The present invention thus relates to prediction of a site on a protein to be modified for improving the function of the protein, and a protein (a thermophilic DNA polymerase) with a modified function based on the prediction.

### BACKGROUND ART

Following the progress of genome analysis and cDNA analysis of various organisms including pathogenic microorganisms, the number of novel genes whose functions are unknown is rapidly increasing, together with the number of proteins encoded by the genes. So far, the analysis of the nucleotide sequence of the whole genome of a microorganism, for example *Mycoplasma genitalium* (Fraser et al., Science 270, 397-403, 1995), *Haemophilus influenzae* (Fleischman et al., Science 269, 496-512, 1995), and *Methanococcus jannaschii* (Bult et al., Science 273, 1058-1073, 1996), has been completed, so that numerous novel proteins predicted from the genome sequence have been discovered. For humans and mice, the cDNA analysis is under way in combination with the genome analysis, which brings about the discovery of a great number of novel proteins.

In such circumstance, prediction of function of a protein with no information on function or a functional site has been a significant issue. If not only a novel protein but also a novel function or a novel functional site of a protein with a known function is discovered, whether or not these proteins are worth industrial or clinical application is possibly determined. Furthermore, such prediction of function possibly enables to prepare a modified protein with a further improved function.

Whether or not a protein encoded by a gene elucidated by genome analysis or cDNA analysis is novel or has a known function has been determined conventionally by searching the homology through protein databases such as Swiss-Prot. So as to predict a functional site, additionally, functionally identical proteins derived from various organisms are extracted from a protein database and are then subjected to alignment, to identify a region conserved in common to them and predict the conserved region as a functional site.

However, disadvantageously, such alignment method cannot be used if a protein obtained by genome analysis or cDNA analysis is an absolutely novel protein. Even if the protein was homologous with known proteins in a protein database, the conserved region occupies most of the amino acid sequence of the protein in case that the protein is homologous to proteins derived from closely related organisms, so that it is impossible to predict the functional site. As to modification of protein, generally, the function of a protein is potentially deteriorated irrespective of the fact that the function is known or unknown once the conserved region is modified, even if the functional site is predicted by alignment. Accordingly, the amino-acid residues outside the conserved region should be modified to improve the function. In other words, it is required to find a novel functional site in such protein to be modified. Using the conventional alignment method, disadvantageously, a novel functional site cannot be discovered or which amino-acid residue should be modified cannot be predicted.

A method for predicting a functional site of a protein with no information on function obtained by genome analysis or cDNA analysis is disclosed herein, together with a system for predicting function of a protein.

Furthermore, a method for predicting a novel functional site of a protein with an unknown function or with a known function and subjecting the functional site to mutation to prepare a modified protein is disclosed.

It is an object of the present invention to provide a protein with a function modified by the method described above.

### DISCLOSURE OF INVENTION

The present invention thus provides a thermophilic DNA polymerase, prepared by artificially modifying the amino acid sequence of Pfu DNA polymerase so that either

(a) the elongation of synthesized DNA chain might not be terminated intermediately during the catalysis for the synthesis of a DNA chain complimentary to a single-stranded DNA, or
(b) the synthesized DNA chain might be more elongated during the catalysis for the synthesis of a DNA chain complimentary to a single-stranded DNA, and wherein the thermophilic DNA polymerase of part (a), comprises the amino acid sequence of SEQ ID No. 1,

and the DNA polymerase of part (b), comprises the amino acid sequence of SEQ ID No. 6 or SEQ ID No. 7.

A first method for predicting a functional site of a protein derived from the entire putative proteins of an organism "a" of which genome data or cDNA data is known, which method comprises the steps of:

(1) calculating the frequency of occurrence of each amino acid and the frequency of occurrence of individual oligopeptides produced by permutations of twenty amino acids in the amino acid sequences of the entire proteins of the organism "a" , and determining the smallest length (n) of oligopeptides satisfying the following criteria;
among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,
(2) calculating in the entire proteins of the organism "a", the frequency of occurrence of the following Aj-oligopeptide of length (n+1), which is a part of the amino acid sequence of length (L) of the protein as a subject for predicting a functional site and contains an amino-acid residue Aj (n+1 ≦j≦L-1) at the j-th position from the N-terminus of the amino acid sequence of the protein;
Aj-oligopeptide: aj1aj2....Aji....ajnaj(n+1)
(wherein, 1≦i≦n+1; Aj=Aji; and Aj is the i-th residue of the oligopeptide),
and calculating in the entire proteins of the organism "a", the frequency of occurrence of the following Xi-oligopeptide of length (n+1);
Xi-oligopeptide: aj1aj2....Xi....ajnaj(n+1)
(wherein, the i-th residue Xi is any amino acid),
(3) calculating ratio Yji of the frequency of occurrence of the Aj-oligopeptide to that of the Xi-oligopeptide,
(4) calculating mean Yj of the Yji;

$$Yj = \sum_{i=1}^{n+1} Yji/(n+1),$$

(5) calculating functional value Zj of Yj;

$$Zj = f(Yj)$$

(wherein, function f is a monotonously decreasing
function or a monotonously increasing function), and defining the Zj value as a representative value of the function of the j-th amino-acid residue Aj of the amino acid sequence of length (L), and
(6) repeating the steps (2) to (5) sequentially and determining the Zj value of each Aj of all the amino-acid residues at the positions between n+1≦j≦L-n in the amino acid sequence of length (L), thereby predicting the degree of the involvement of each amino-acid residue in the function of the protein by using the dimension of the Zj value as an indicator is disclosed herein.

A second method for predicting a functional site of a protein derived from the entire putative proteins of an organism "a" of which genome data or cDNA data is known, which method comprises the steps of:

(1) calculating the frequency of occurrence of each amino acid and the frequency of occurrence of individual oligopeptides produced by permutations of twenty amino acids in the amino acid sequences of the entire proteins of the organism "a",
(2) as to a protein of the organism "a",
(2') calculating in the entire proteins of the organism "a", the frequency of occurrence of the following Aj-oligopeptide of given length of (n) (1≦n≦M, provided that M is the the smallest length of oligopeptides satisfying the criterion that all the oligopeptides of length M are at frequency 1 of the occurrence), which Aj-oligopeptide is a part of the amino acid sequence of length (L) of the protein and contains an amino-acid residue Aj (n≦j≦L-n+1) at the j-th position from the N-terminus of the amino acid sequence of the protein;
Aj-oligopeptide: aj1aj2....aji....ajn

(wherein, $1 \leqq i \leqq n+1$; Aj=aji, and Aj is the i-th residue of the oligopeptide), and calculating in the entire proteins of the organism "a", the frequency of occurrence of the following Xi-oligopeptide of length (n) corresponding to the length of Aj-oligopeptide;
Xi-oligopeptide: aj1aj2....Xi....ajn
(wherein, the i-th residue Xi is any amino acid),
(3) calculating ratio Yji of the frequency of occurrence of the Aj-oligopeptide to that of the Xi-oligopeptide,
(4) calculating mean Y(j,n) of the Yji;

$$Y(j,n) = \sum_{i=1}^{n} Yji/n,$$

(5) calculating functional value Z(j,n) of Y(j,n);

$$Z(j,n) = -\log(Y(j,n)),$$

(6) repeating the steps (2') to (5) sequentially and determining the Z(j,n) value of each amino-acid residue Aj at the j-th position ($n \leqq j \leqq L-n+1$) in the amino acid sequence of length (L),
(7) sequentially repeating the steps (2) to (6) for the entire proteins of the organism "a", thereby determining the distribution of the Z(j,n) value of each amino-acid residue in the entire proteins, and the Z(j,n) values are classified into twenty according to the twenty amino acids, and then calculating mean Av(Aa) of the Z(j,n) values for each amino acid Aa and the standard deviation Sd (Aa) of the distribution thereof, on the basis of the distribution, to calculate a function g to the j-th amino-acid residue Aj of a protein for normalizing the difference in distribution due to the species of amino-acid residues;

$$g = (Z(j,n),\ Aj) = [Z(j,n) - Av(Aa)]/Sd(Aa)$$

(wherein, Aj = Aa),
(8) calculating a value D(j,n) of the function g of each Aj of all the amino-acid residues at the j-th position ($n \leqq j \leqq L-n+1$) of a protein in the entire proteins as recovered in the step (7);

$$D(j,n) = g(Z(j,n),\ Aj),.$$

and
(9) defining the representative value of the function of the j-th amino-acid residue in the amino acid sequence of length (L) as a functional value Wj of the Z(j,n) and D(j,n);

$$Wj=h(Z(j,1),Z(j,2),..,Z(j,M),D(j,1),D(j,2),..,D(j,M))$$

thereby predicting the degree of the involvement of each amino-acid residue in the function of the protein by using the dimension of the Wj value as an indicator is further disclosed.

A system for automatically conducting the first method has been developed, at least comprising the following units (a) to (g);

(a) an outer memory unit memorizing the amino acid sequence data of the entire putative proteins of organism "a" of which genome data or cDNA data is known, as well as an existing protein data base,
(b) a calculation/memory unit, composed of CPU calculating the frequency of occurrence of each amino acid and the frequency of occurrence of individual oligopeptides produced by permutations of twenty amino acids, in the amino acid sequences of the entire proteins of the organism "a", and a memory unit having the memory of the calculation results,

(c) a calculation/memory unit, composed of CPU calculating the smallest length (n) of oligopeptides satisfying the following criteria among the individual oligopeptides of which the frequencies of the occurrences being memorized in the unit (b) ;

among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,

and a memory unit having the memory of the (n),

(d) a calculation/memory unit, composed of CPU calculating in the entire proteins of the organism "a", the frequency of occurrence of the following Aj-oligopeptide of length (n+1), which is a part of the amino acid sequence of length of (L) of the protein as a subject for predicting a functional site and contains an amino-acid residue Aj ($n+1 \leqq j \leqq L-n$) at the j-th position from the N-terminus of the amino acid sequence of the protein;

Aj-oligopeptide: aj1aj2....Aji....ajnaj(n+1)

(wherein, $1 \leqq i \leqq n+1$; Aj = Aji; and Aj is the i-th residue of the oligopeptide),

and calculating in the entire proteins of the organism "a", the frequency of occurrence of the following Xi-oligopeptide of length (n+1);

Xi-oligopeptide: aj1aj2....Xji....ajnaj(n+1)

(wherin, the i-th residue Xji is any amino acid, and a memory unit having the memory of the calculation results,

(e) a calculation/memory unit, composed of CPU calculating ratio Yji of the frequency of occurrence of the Aj-oligopeptide to that of the Xi-oligopeptide, and a memory unit having the memory of Yji,

(f) a calculation/memory unit, composed of CPU calculating mean Yj of the Yji;

$$Yj = \sum_{i=1}^{n+1} Yji/(n+1),$$

and a memory unit having the memory of Yj, and

(g) a calculation/memory unit, composed of CPU calculating functional value Zj of Yj;

$$Zj = f(Yj)$$

(wherein, function f is a monotonously decreasing function or a monotonously increasing function),

and a memory unit having the memory of Zj.

Further a system for automatically conducting the second method has been developed, at least comprising the following units (a) to (i) ;

(a) an outer memory unit memorizing the amino acid sequence data of the entire putative proteins of organism "a" of which genome data or cDNA data is known, as well as an existing protein data base,

(b) a calculation/memory unit, composed of CPU calculating the frequency of occurrence of each amino acid and the frequency of occurrence of individual oligopeptides produced by permutations of twenty amino acids in the amino acid sequences of the entire proteins of the organism "a", and a memory unit having the memory of the calculation results,

(c) a calculation/memory unit, composed of CPU calculating in the entire proteins of the organism "a", the frequency of occurrence of the following Aj-oligopeptide of given length of (n) ($1 \leqq n \leqq M$, provided that M is the smallest length of oligopeptides satisfying the criterion that all the oligopeptides of length M are at frequency 1 of the occurrence), which Aj-oligopeptide is a part of the amino acid sequence of length (L) of a given protein of the organism "a" and contains an amino-acid residueAj ($n \leqq j \leqq L-n+1$) at the j-thposition from N-terminus of the amino acis sequene of the protein;

Aj-oligopeptide: aj1aj2....aji....ajn

(wherein, $1 \leqq i \leqq n+1$; Aj=aji, and Aj is the i-th residue of the oligopeptide),

and calculating in the entire proteins of the organism "a", the frequency of occurrence of the following Xi-oligopeptide of length (n) corresponding to the length of Aj-oligopeptide;
Xi-oligopeptide: aj1aj2....Xji....ajn
(wherein, the i-th residue Xji is any amino acid),
and a memory unit memorizing the calculation results,
(d) a calculation/memory unit, composed of CPU calculating ratio Yji of the frequency of occurrence of theAj-oligopeptide to that of the Xi-oligopeptide, and a memory unit having the memory of the Yji,
(e) a calculation/memory unit, composed of CPU calculating mean Y(j,n) of the Yji;

$$Y(j,n) = \sum_{i=1}^{n} Yji/n,$$

and a memory unit having the memory of Y(j,n),
(f) a calculation/memory unit, composed of CPU calculating functional value Z(j,n) of Y(j,n);

$$Z(j,n) = -\log(Y(j,n)),$$

and a memory unit having the memory of Z(j,n),
(g) a calculation/memory unit, composed of CPU calculating the Z(j,n) value of each amino-acid residue in the amino acid sequences of the entire proteins of the organism "a", calculating the distribution of the Z(j,n) value of each amino-acid residue in the entire proteins, and the Z(j,n) values are classified into twenty according to the twenty amino acids, and then caclulating mean Av(Aa) of the Z(j,n) values for each amino acid Aa and the standard deviation Sd (Aa) of the distribution thereof, on the basis of the distribution, to determine function g for normalizing the difference in distribution due to the species of amino-acid residues;

$$g = (Z(j,n),\ Aj) = [Z(j,n) - Av(Aa)]/Sd(Aa)$$

(wherein, Aj = Aa)
and a memory unit having the memory of g,
(h) a calculation/memory unit, composed of CPU calculating value D (j,n) of function g memorized in the unit (g) concerning each of all the amino-acid residues Aj at the j-th position (n≦j≦L-n+1) in the amino acid sequence of length (L) ;

$$D(j,n) = g(Z(j,n),\ Aj)$$

and a memory unit having the memory of the D(j, n) value, and
(i) a calculation/memory unit, composed of a calculation unit calculating an appropriate functional value Wj of the Z (j,n) and D(j,n) of each amino-acid residue in the amino acid sequence;

$$Wj=h(Z(j,1),Z(j,2),\ldots,Z(j,M),D(j,1),D(j,2),\ldots,D(j,M))$$

and a memory unit having the memory of the Wj value.

A third method is disclosed which is a method for modifying the known function of protein "A" derived from the entire proteins of organism "a" of which genome data or cDNA data has been known, which method comprises the steps of:

(1) extracting a protein closely related to the protein "A" from an existing protein data base and subjecting the proteins to alignment,
(2) calculating the frequency of occurrence of each amino acid and the frequency of occurrence of individual oligopeptides produced by permutations of twenty amino acids in the amino acid sequences of the entire proteins of the organism "a", and determing the smallest length (n) of oligopeptides satisfying the following criteria;

among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,

(3) calculating in the entire proteins of the organism "a", the frequency of occurrence of the following Aj-oligopeptide of length (n+1), which is a part of the amino acid sequence of length (L) of the protein "A" and contains an amino-acid residue Aj ($n+1 \leqq j \leqq L-n$) at the j-th position from the N-terminus of the amino acid sequence of the protein;

Aj-oligopeptide: aj1aj2....Aji....ajnaj(n+1)

(wherein, $1 \leqq i \leqq n+1$; Aj=Aji; and Aj is the i-th residue of the oligopeptide),

and calculating in the entire proteins of the organism "a", the frequency of occurrence of the following Xi-oligopeptide of length (n+1);

Xi-oligopeptide: aj1aj2....Xji....ajnaj(n+1)

(wherein, the residue Xji is any amino acid),

(4) calculating ratio Yji of the frequency of occurrence of the Aj-oligopeptide to that of the Xi-oligopeptide,

(5) calculating mean Yj of the Yji;

$$Yj = \sum_{i=1}^{n+1} Yji/(n+1),$$

(6) calculating functional value Zj of Yj;

$$Zj = f(Yj)$$

(wherein, function f is a monotonously decreasing function or a monotonously increasing function),

and defining the Zj value as a representative value of the function of the j-th amino-acid residue of the amino acid sequence of length (L) of the protein "A",

(7) sequentially repeating the steps (3) to (6) and determining the Zj value of each of all the amino-acid residues at position between $n+1 \leqq j \leqq L-n$ in the amino acid sequence of length (L),

(8) selecting at least one amino-acid residue to be subjected to mutation on the basis of the alignment data carried out in the step (1) from the amino acid sequence of length (L) of the protein "A", sequentially repeating the steps (3) to (6) for variant amino-acid residues in various mutated amino acid sequences where the selected amino-acid residue has been mutated into another amino-acid residue, to determine the Zj value of the variant amino-acid residues,

(9) selecting a mutated amino acid sequence wherein the Zj value of the variant amino-acid residue as determined in the step (8) is larger or smaller than the Zj value of the wild type amino-acid residue as determined in the step (7), and

(10) preparing a modified gene encoding the modified amino acid sequence from the protein "A" gene, and producing the modified protein as the expression product of the gene.

Further a fourth method is disclosed which is a method for modifying the function of protein "B" derived from an organism "b" of which genome data or cDNA data has been unknown, which method comprises the steps of:

(1) extracting protein "A" most closely related to protein "B" from the entire proteins of organism "a" of which genome data or cDNA data being known and subjecting the protein to alignment, or extracting a protein closely related to protein "B" from an existing protein data base to subject the protein to alignment,

(2) calculating in the amino acid sequences of the entire proteins of the organism "a", the frequency of occurrence of each amino acid and the frequency of occurrence of individual oligopeptides produced by permutations of twenty amino acids, and determining the smallest length (n) of oligopeptides satisfying the following criteria;

among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,

(3) calculating in the entire proteins of the organism "a", the frequency of occurrence of the following Aj-oligopeptide of length (n+1), which is a part of the amino acid sequence of length of (L) of the protein "A" and contains an amino-acid residue Aj (n+1≦j≦L-n) at the j-th position from the N-terminus of the amino acid sequence of the protein;
Ai-oligopeptide: aj1aj2....Aji....ajnaj(n+1)
(wherein, 1≦i≦n+1; Aj=Aji; and Aj is the i-th residue of the oligopeptide),
and calculating in the entire proteins of the organism "a", the frequency of occurrence of the following Xi-oligopeptide of length (n+1);
Xi-oligopeptide: aj1aj2....Xji....ajnaj (n+1)
(wherein, the i-th residue Xji is any amino acid),
(4) calculating ratio Yji of the frequency of occurrence of the Aj-oligopeptide to that of the Xi-oligopeptide,
(5) calculating mean Yj of the Yji;

$$Yj = \sum_{i=1}^{n+1} Yji/(n+1),$$

(6) calculating functional value Zj of Yj;

$$Zj = f(Yj)$$

(wherein, function f is a monotonously decreasing function or a monotonously increasing function) ,
and defining the Zj value as a representative value of the function of the j-th amino-acid residue Aj of the amino acid sequence of length (L) of the protein "A",
(7) sequentially repeating the steps (3) to (6) and determining the Zj value of each of all the amino-acid residues at position between n+1≦j≦L-n in the amino acid sequence of length (L),
(8) selecting at least one amino-acid residue to be subjected to mutation on the basis of the alignment data carried out in the step (1) from the amino acid sequence of length (L) of the protein "A", sequentially repeating the steps (3) to (6) for variant amino-acid residues in various mutated amino acid sequences where the selected amino-acid residue has been mutated into another amino-acid residues, to determine the Zj value of the variant amino-acid residues,
(9) selecting the mutation position and the mutated amino-acid residue wherein the Zj value of the variant amino-acid residue as determined in the step (8) is larger or smaller than the Zj value of the wild type amino-acid residue as determined in the step (7), and
(10) preparing a modified gene encoding the modified amino acid sequence having the mutated amino-acid residue at the position from the protein "B" gene, and producing the modified protein as the expression product of the gene.

A fifth method is also disclosed which is a method for modifying the known function of protein "A" derived from the entire proteins of organism "a" of which genome data or DNA data has been known, which method comprises the steps of:

(1) extracting proteins closely related to the protein "A" from an existing protein data base and subjecting the proteins to alignment,
(2) calculating the frequency of occurrence of each amino acid and the frequency of occurrence of individual oligopeptides produced by permutations of twenty amino acids, in the amino acid sequences of the entire proteins of the organism "a",
(3) as to a protein of the organism "a",
(3') calculating in the entire proteins of the organism "a", the frequency of occurrence of the following Aj-oligopeptide of given length of (n) (1≦n≦M, provided that M is the smallest length of oligopeptides satisfying the criterion that all the oligopeptides of length M are at frequency 1 of the occurrence), which Aj-oligopeptide is a part of the amino acid sequence of the protein and contains an amino-acid residue Aj (n≦j≦L-n+1) at the j-th position from the N-terminus of the protein;
Aj-oligopeptide: aj1aj2....aji....ajn
(wherein, 1≦i≦n; Aj=aji, and Aj is the i-th residue of the oligopeptide),
and calculating in the entire proteins of the organism "a", the frequency of occurrence of the following Xi-oligopeptide of length (n) corresponding to the length of Aj-oligopeptide;

Xi-oligopeptide: aj1aj2....Xji....ajn
(wherein, the i-th residue Xji is any amino acid),
(4) calculating ratio Yji of the frequency of occurrence of the Aj-oligopeptide to that of the Xi-oligopeptide,
(5) calculating mean Y(j,n) of the Yji;

$$Y(j,n) = \sum_{i=1}^{n} Yji/n,$$

(6) calculating functional value Z(j,n) of Y(j,n);

$$Z(j,n) = -\log(Y(j,n)),$$

(7) repeating the steps (3') to (6) sequentially and determining the Z(j,n) value of each amino-acid residue Aj at position j (n≦j≦L-n+1) in the amino acid sequence of length (L) ,
(8) sequentially repeating the steps (3) to (7) for the entire proteins of the organism "a", thereby determining the distribution of the Z(j,n) value of each amino-acid residue in the entire proteins, and the Z(j,n) values are classified into twenty according to the twenty amino acids, and then calculating mean Av (Aa) of the Z(j,n) values for each amino acid Aa and the standard deviation Sd (Aa) of the distribution thereof, on the basis of the distribution, to determine function g to the j-th amino-acid residue Aj of a protein for normalizing the difference in distribution due to the species of amino-acid residues;

$$g = (Z(j,n),\ Aj) = [Z(j,n) - Av(Aa)]/Sd(Aa)$$

(wherein, Aj = Aa),
(9) calculating value D(j,n) of the function g of each Aj of all the amino-acid residues at the j-th position (n≦j≦L-n+1) of a protein in the entire proteins as recovered in the step (8);

$$D(j,n) = g(Z(j,n),\ Aj),$$

(10) defining the representative value of the function of the j-th amino-acid residue in the amino acid sequence of length (L) as functional value Wj of the Z(j,n) and D(j,n);

$$Wj=h(Z(j,1),Z(j,2),..,Z(j,M),D(j,1),D(j,2),..,D(j,M))$$

(11) sequentially repeating the steps (3) to (10), to determine the individual Wj values of all the amino-acid residues at the position (n+1≦j≦L-n) in the amino acid sequence of length (L),
(12) selecting at least one amino-acid residue to be subjected to mutation on the basis of the alignment data carried out in the step (1) from the amino acid sequence of length (L) of the protein "A", and sequentially repeating the steps (3) to (10) for variant amino-acid residues in various mutated amino acid sequences where the selected amino-acid residue has been mutated into another amino-acid residue, to determine the Wj value of the variant amino-acid residue,
(13) selecting a mutated amino acid sequence wherein the Wj value of the variant amino-acid residue as determined in the step (12) is larger or smaller than the Wj value of the wild type amino-acid residue as determined in the step (10), and
(14) preparing a modified gene encoding the modified amino acid sequence from the protein "A" gene, and producing the modified protein as the expression product of the gene.

As indicated above, the present invention relates to a thermophilic DNA polymerase, prepared by artificially modifying the amino acid sequence of *Pfu* DNA polymerase so that the elongation of synthesized DNA chain might not be terminated intermediately during the catalysis for the synthesis of a DNA chain complimentary to a single-stranded DNA. A thermophilic DNA polymerase comprising the amino acid sequence of SQ ID No.1 is provided by the invention.

In association with the invention, the present application provides a DNA sequence encoding the amino acid sequence of SQ ID No.1 and a recombinant vector carrying the DNA sequence. Such recombinant vector includes recombinant plasmid pDP320 carried on *Escherichia coli* HMS174 (DE3) /pDP320 (FERM P-16052).

Still furthermore, a method for preparing a thermophilic DNA polymerase is disclosed, comprising culturing a cell transformed with an expression vector carrying the DNA sequence and isolating and purifying the objective enzyme generated in a culture medium.

The present invention further relates to a thermophilic DNA polymerase, prepared by artificially modifying the amino acid sequence of *Pfu* DNA polymerase so that the synthesized DNA chain might be more elongated during the catalysis for the synthesis of a DNA chain complimentary to a single-stranded DNA.

A thermophilic DNA polymerase comprising the amino acid sequence of SQ ID No. 6 or a DNA polymerase comprising the amino acid sequence of SQ ID No.7 are provided by the invention.

In association with this aspect of the present invention, the present application provides a DNA sequence encoding the amino acid sequence of SQ ID No. 6 or 7, and recombinant vectors carrying such DNA sequences, respectively. As such vectors, there are provided recombinant plasmid pDP5b17 carried on *Escherichia coli* HMS174 (DE3)/pDP5b17 (FERM BP-6189) (vector carrying the DNA sequence encoding the amino acid sequence of SQ ID No. 1), and recombinant plasmid pDP5C4 carried on *Escherichia coli* HMS174 (DE3) /pDP5C4 (FERM BP-6190) (vector carrying the DNA sequence encoding the amino acid sequence of SQ ID No.1).

Still furthermore, a method for producing a DNA polymerase is disclosed, : comprising culturing a cell transformed with an expression vector carrying the DNA sequence and isolating and purifying the objective enzyme produced in a culture medium.

The first method for predicting a protein functional site disclosed herein has been established on a principle as follows. A protein is composed of a sequence of twenty amino acids, but the sequence is not random. Hence, the frequency of occurrence of a specific oligopeptide as a partial amino acid sequence in the entire proteins encoded by genome derived from an appropriate organism species is not constant, but some oligopeptides occur at high frequencies in various proteins while other oligopeptides rarely occur therein. It is recognized that among them, oligopeptides highly frequently occurring in common to various proteins do not have any potency to determine the uniqueness (specificity) of individual proteins, namely any potency to determine the functions, while oligopeptides occurring at low frequencies adversely determine the uniqueness and functions of individual proteins.

It is suggested that the functional site of protein is composed of oligopeptides occurring at low frequencies. Additionally, longer oligopeptides, more rarely occurring, increase in number. In other words, oligopeptide of length (n+1) as shown in the step (3) according to the first method

is mostly the shortest oligopeptide occurring at a low frequency, and the calculated functional value Zj of amino-acid residue Aj at an appropriate position j in the oligopeptide is the coefficient of the occurrence (namely, the representative value of the function) of the amino-acid residue Aj at the position.

According to the second method for predicting the protein functional site disclosed herein, the contribution degree of the amino-acid residue Aj to the frequency of the occurrence of Aj-oligopeptide can be evaluated on the basis of the ratio Yji of the frequency of the occurrence of the Aj-oligopeptide to that of the Xi-oligopeptide as shown in the step (3), and thus, the calculated functional value Z(j,n) of the amino-acid residue Aj at an appropriate position of a protein serves as the coefficient of the occurrence of the amino-acid residue Aj at the position (namely, the representative value of the function).

Furthermore, the value Z(j, n) varies, depending on the species of amino-acid residue Aj. In the step (7) according to the method, the distribution of the Z(j, n) value of each of twenty amino acids is determined in the entire proteins of organism "a", to determine D(j, n) value by normalizing the Z(j, n) value on the basis of the mean and standard deviation of Z(j,n) value of each amino acid, as determined on the basis of the distribution, which serves as the representative value of the function, after correction of the bias due to each amino-acid residue species.

Furthermore, longer oligopeptides, more rarely occurring, increase in number. Because the Z (j, n) and D(j, n) values generally vary, depending on the length (n), accordingly, the functional value Wj of the Z(j, n) and D(j, n) as determined on a variety of length (n) is defined as the representative value of the function.

The systems for predicting a protein functional site are individually systems for automatically carrying out the first and second methods; the third and fourth methods discussed above which are methods for modifying protein are methods for preparing mutant proteins by substituting the amino-acid residue at the functional site predicted by the first method with another amino-acid residue Still further, the fifth method disclosed above which is a method for modifying a protein is a method for preparing a mutant protein by substituting the amino-acid residue at the functional site predicted by the second method with another amino-acid residue. In accordance with the present invention, a novel thermophilic DNA polymerase of SEQ ID No. 1, 6 or 7 is provided as a modified protein.

The term "DNA polymerase" is the generic name of enzymes catalyzing the synthesis of a DNA chain complimentary to a single-stranded DNA. DNA polymerase is an essential enzyme for DNA sequencing and *in vitro* DNA amplification, and "thermophilic DNA polymerase" is inevitable for PCR (polymerase chain reaction) in terms of the automation of a series of the reaction cycles.

Such thermophilic DNApolymerase includes known ones, for example *Taq*, *Pfu, KOD*, which are separately used, depending on the characteristic performance. *Pfu* DNA polymerase in particular has been known as an enzyme with an extremely low frequency of erroneous reading during the synthesis of DNA strands (at a high fidelity). However, the *Pfu* DNA polymerase is inappropriate for the amplification of polymeric DNAs such as genome DNA, because the synthetic DNA yielded by the *Pfu* DNA polymerase is low and the activity thereof to elongate a synthetic chain is insufficient. Thus, a novel *Pfu* DNA polymerase prepared according to the third method is disclosed.

BRIEF DESCRIPTION OF DRAWINGS

Fig.1 depicts graphically the individual frequencies of the occurrences of oligopeptides of lengths 3, 4 and 5, and the distributions of the individual frequencies thereof according to the first method;

Fig.2 depicts an example of an amino acid sequence of a length of 20, and examples of Aj-oligopeptide of a length of 4, containing the amino-acid residue Met at position 5 in the sequence and examples of Xi-oligopeptides;

Fig.3 depicts graphically the individual frequencies of the occurrences of oligopeptides of lengths of 2, 3, 4 and 5, and the distributions of the individual frequencies thereof according to the second method;

Fig.4 depicts an example of the flow chart for conducting the step (1) of the second method;

Fig.5 depicts an example of the flow chart for conducting the steps (2') to (3) of the second method;

Fig.6 depicts an example of the flow chart for conducting the steps (4) to (5) of the second method;

Fig.7 depicts a distribution of the frequency of Z (j, 3) of each of three amino acids, see below, according to the second method, wherein the solid line expresses the distribution of that of isoleucine (Ile); the dotted line expresses the distribution of that of alanine (Ala); and the alternate long and short dash line expresses the distribution of that of methionine (Met);

Fig.8 depicts an example of the flow chart for conducting the step (7) of the second method;

Fig.9 depicts an example of the flow chart for conducting the step (8) of the second method;

Fig.10 depicts an example of the flow chart for conducting the step (9) of the second method;

Fig.11 depicts a block diagram illustrating the first system disclosed above;

Fig.12 depicts a block diagram illustrating the first system disclosed above;

Fig.13 depicts the electrophoresis results of conventional *Pfu* DNA polymerase and KOD DNA polymerase, indicating the primer elongating activities thereof;

Fig.14 depicts a distribution chart of the plotted Zj = -logYj value for the whole amino acid sequence of the α-type DNA polymerase encoded by MJ0885, which value is calculated by the first method;

Fig.15 depicts a distribution chart of the plotted Zj =
-logYj value for partial sequences (motif A and motif C) of the amino acid sequence of which the distribution chart is shown in Fig.14;

Fig.16 depicts a frequency distribution chart of the Zj = -logYj value calculated on the basis of the amino acid sequence of the α-type DNA polymerase encoded by MJ0885;

Fig.17 depicts alignment charts of the amino acid sequences of the individual motif Cs from the α-type DNA polymerases *Pfu, KOD* and *MJ*;

Fig.18 depicts distribution charts of the plotted Zj = -logYj values for the individual motif Cs of the α-type DNA polymerases *Pfu*, *KOD* and *MJ*;

Fig.19 depicts distribution charts of the plotted values of Wj = Z(j, 3) - Z(j,1) (in solid line), Wj = Z(j, 4) - Z(j, 3) (in dotted line) and Wj = Z(j, 5) - Z (j, 3) (in alternate long and short dash line) of the 100 residues from the N-terminus of the whole amino acid sequence of the α-type DNA polymerase encoded by MJ0885, and these values are calculated by the second method;

Fig.20 depicts distribution charts of the plotted value Wj =Z(j, 5) - Z (j, 3) for partial sequences (regions comprising exoI, exoII, motif A, motif B and motif C) of the amino acid sequence of the α-type DNA polymerase encoded by MJ0885;

Fig.21 depicts distribution charts of the plotted values of Wj = D(j, 3) (in dark color) and Wj = D (j, 5) (in pale color) for partial sequences (regions comprising exoI, exoII, motif A, motif B and motif C) of the amino acid sequence of the α-type DNA polymerase encoded by MJ0885;

Fig.22 depicts distribution charts in dark color of the positions of amino-acid residues with Wj = D (j, 3) of 2 or more or of 2 or less in the three-dimensional structure of the amino acid sequence of enolase encoded by MJ0232 on a three-dimensional structure model;

Fig.23 depicts the results of electrophoresis, indicating the primer elongating activities of the conventional *Pfu* DNA polymerase (wild type) and the modified *Pfu* DNA polymerase I of the present invention; and

Fig.24 depicts the results of electrophoresis, indicating the primer elongating activities of the conventional *Pfu* DNA polymerase (wild type) and the modified *Pfu* DNA polymerases II and III of the present invention.

BEST MODE FOR CARRYING OUT THE INVENTION

The first method for predicting a functional site of a protein is a method for predicting the functional site of a protein of organism "a" with a known genome data or cDNA analysis data, in the entire putative proteins of the organism "a", essentially comprising the following steps (1) to (6).

Step (1):

By calculating the frequency of the occurrence of each amino acid and the frequencies of the occurrences of individual oligopeptides produced by permutations of twenty amino acids in the amino acid sequences of the entire proteins of the organism "a", the oligopeptide length (n) is determined.

The length (n) is determined, then, as the smallest integer satisfying the following criteria:

"Among oligopeptides of length (n), the number of oligopeptides that occur once in the entire proteins is smaller than the number of oligopeptides that occur twice in the entire proteins; among oligopeptides of length (n+1), the number of oligopeptides that occur once in the entire proteins is larger than the number of oligopeptides that occur twice in the entire proteins."

For example, Fig.1 depicts distribution charts of the frequencies of the occurrences of oligopeptides of a length of 3, oligopeptides of a length of 4 and oligopeptides of a length of 5, in the entire proteins encoded by the genome of a microorganism *Methanococcus jannaschii* (Bult et al., Science 273, 1053-1073, 1996). In the case of the three types of length of the oligopeptides shown in Fig.1, the smallest n in the step (1) is 3.

Step (2) :

Given that the j-th amino-acid residue from the N-terminus of the amino acid sequence of length (L) of the protein as a subject for predicting a functional site is described here as Aj (n+1≦j≦L-n), the frequency of occurrence of a partial sequence of the amino acid sequence of the protein, which sequence corresponds to the following Aj-oligopeptide of length (n+1), containing the j-th amino-acid residue Aj;

Aj-oligopeptide: ajlaj2....Aji....ajnaj(n+1)

(wherein, 1≦i≦n+1; Aj =Aji; and Aj is the i-th residue of the oligopeptide),

and the frequency of the occurrence of the following Xi-oligopeptide of length (n+1);

Xi-oligopeptide: aj1aj2....Xji....ajnaj(n+1)

(wherein, the i-th residue Xji is any amino acid) should be determined in the entire proteins of the organism "a".

Such Aj-oligopeptide and Xi-oligopeptide can be illustrated for example in Fig.2. The upper row (1) in Fig.2 expresses in single letter code the partial sequence from the N-terminus to the 20-th amino-acid residue of the putative amino acid sequence speculated from the gene MJ0885, which is believed to encode the α-type DNA polymerase of *Methanococcus jannaschii* (Bult et al., Science 273, 1058-1073, 1996); the middle row (2) expresses examples of Aj-oligopeptide of a length of 4, containing the 5-th amino-acid residue Met (M) in the amino acid sequence; and the rows (3) to (6) further below express examples of Xi-oligopeptide containing the 5-th amino-acid residue M.

Step (3) :

Calculating ratio Yji of the frequency of the occurrence of the Aj-oligopeptide to that of the Xi-oligopeptide.

Step (4):

The mean Yj of the Yji is calculated as follows.

$$Yj = \sum_{i=1}^{n+1} Yji \; /(n+1),$$

Step (5):

Monotonously decreasing functional value or monotonously increasing functional value Zj of Yj is determined as follows;

$$Zj = f(Yj).$$

The Zj value is defined as a representative value of the function of the j-th amino-acid residue of the amino acid sequence of length (L).

Step (6):

By subsequently repeating the steps (2) to (5) sequentially and determining the Zj value of each of all the amino-acid residues at position n+1≦j≦L-n, the degree of the involvement of each amino-acid residue in the function of the protein is predicted by using the dimension of the Zj value as an indicator. More specifically, because the manner of occurring of each amino-acid residue in the context is expressed as the functional value Zj of Yj, a larger Zj value indicates a lower frequency of occurrence of the amino-acid residue if Zj is a monotonously decreasing functional value, which suggests that the amino-acid residue has higher responsibility over the performance of the function. If Zj is a monotonously increasing function, additionally, it is suggested that an amino-acid residue with a smaller Zj value has greater responsibility over the function.

By expressing the Zj value of each amino-acid residue for example in a distribution chart wherein the Zj value is plotted on the vertical axis while the amino acid sequence is shown on the horizontal axis, furthermore, the functional site can be confirmed at a glance.

The second method is a method for predicting a functional site of an appropriate protein in the entire putative proteins of the organism "a" with a known genome data or cDNA analysis data, essentially comprising the following steps (1) to (9).

Step (1) :

The frequency of the occurrence of each amino acid and the frequencies of the occurrences of individual oligopeptides produced by permutations of twenty amino acids, in the amino acid sequences of the entire proteins of the organism "a", are calculated.

For example, Fig.3 shows a distribution chart of the frequencies of the occurrences of oligopeptides of a length of 3, oligopeptides of a length of 4 and oligopeptides of a length of 5, which are determined in the entire proteins encoded by the genome of a microorganism *Methanococcus jannaschii* (Bult et al., Science 273, 1058-1073, 1996) on the basis of the genome data of the microorganism.

Fig.4 depicts an example of the flow chart for carrying out the step (1).

Step (2):

As to a protein of organism "a",

Step (2') :

given that the j-th amino-acid residue from the N-terminus of the amino acid sequence of length (L) of the protein is described here as Aj, the frequency of the occurrence of a partial sequence of the amino acid sequence of the protein, which sequence corresponds to the following Aj-oligopeptide of an appropriate length n (1≦n≦M, provided that "M" is the smallest length of oligopeptides satisfying the following criterion; all the oligopeptides of length M are at frequency 1 of the occurrence), containing the j-th amino-acid residue Aj (n≦j≦L-n+1);
Aj-oligopeptide: aj1aj2....Aji....ajn

(wherein, 1≦i≦n; Aj = Aji and Aji is the i-th residue of the oligopeptide),
and the frequency of the occurrence of the following Xi-oligopeptide of the length n corresponding to the legnth of the Aj-oligopeptide;
Xi-oligopeptide: aj1aj2....Xji....ajn
(wherein, the i-th residue Xji is any amino acid), are calculated in the entire proteins of the organism "a".
In the same manner as by the first method, such Aj-oligopeptide and Xi-oligopeptide are for example illustrated as in Fig.2.

Step (3):

The ratio Yji of the frequency of the occurrence of the Aj-oligopeptide to that of the Xi-oligopeptide is calculated.
Fig.5 depicts an example of the flow chart for carrying out the aforementioned steps (2') to (3).

Step (4):

The mean Y(j, n) of the Yji is calculated as described below:

$$Y(j,\ n) = \sum_{i=1}^{n} Yji/n.$$

Step (5) :

The logarithmic value Z(j, n) of Y(j, n) is determined as follows.

$$Z(j,\ n) = -\log(Y(j,\ n))$$

Fig.6 depicts an example of the flow chart for carrying out the aforementioned steps (4) to (5).

Step (6):

By subsequently repeating the steps (2') to (5) sequentially, the Z(j, n) value of each of all the amino-acid residues at position n≦j≦L-n+1 in the amino acid sequence of length (L) is determined.

Step (7):

By sequentially repeating the steps (2) to (6) over the entire proteins of the organism "a", thereby determining the distribution of the Z(j,n) value of each amino-acid residue in the entire proteins, and the Z(j,n) values are classified into twenty according to the twenty amino acids, and then calculating mean Av(Aa) of the Z(j,n) values for each amino acid Aa and the standard deviation Sd (Aa) of the distribution thereof, on the basis of the distribution, to determine function g to the j-th amino-acid residue Aj of a protein for normalizing the difference in distribution due to the species of amino-acid residues is determined;

$$g = (Z(j,\ n),\ Aj) = [Z(j,\ n) - Ad(Aa)]/Sd(Aa)$$

(whrerein, Aj = Aa).
For example, Fig. 7 depicts a distribution of the frequency of Z (j, n) for three species of amino acids, namely isoleucine (Ile), alanine (Ala) and methionine (Met), in the entire proteins encoded by the genome of *Methanococcus jannaschii* (Bult et al., Science 273, 1058-1073, 1996). Based on the distribution, the mean and standard deviation of the Z(j, n) values for an amino acid isoleucine (Ile), namely Ad (Ile) and Sd(Ile), respectively, are determined as Ad(Ile) = 3.16 and Sd(Ile) = 0.17, and the function g for Aj = Ile is determined as follows.

$$g= (Z(j, n), Aj) = (Z(j, n) - 3.16)/0.17$$

Fig.8 depicts an example of the flow chart for carrying out the step (7).

Step (8):

The value D(j, n) of the function g of each of all the amino-acid residues Aj at position n≦j≦L-n+1 in the amino acid sequence of length (L) as recovered in the step (7) is determined;

$$D(j, n) = g (Z(j, n), Aj).$$

Fig.9 depicts an example of the flow chart for carrying out the step (8).

Step (9) :

The functional value Wj of the Z(j, n) value and the D(j, n) value is determined as follows.

$$Wj = h(Z(j, 1), Z(j, 2) ,..., Z(j, M), D(j,1), D(j, 2),..., D(j, M))$$

By defining the value of the Wj as the representative value of the function of the j-th amino-acid residue in the amino acid sequence of length (L), the degree of the responsibility of each amino-acid residue over the function of the protein is estimated by using the dimension of the Wj value as an indicator.
Fig.10 depicts an example of the flow chart for carrying out the step (9). By expressing the Wj value of each amino-acid residue for example in a distribution chart wherein the Wj value is plotted on the vertical axis while the amino acid sequence is shown on the horizontal axis, furthermore, the functional site can be confirmed at a glance.
If the three-dimensional structure of the protein as a subject for predicting the functional site is known or if a three-dimensional structure model thereof can be prepared by known methods (for example, homology modeling method, Peitsch, Proceedings of the Fifth International Conference on Intelligent Systems for Molecular Biology, 1997, 5, 234-236), the distribution is expressed on the three-dimensional structure, whereby a spatial arrangement of an amino-acid residue as a candidate of a novel functional site can be confirmed.
A system for automatically carrying out the method for predicting a functional site in accordance with the first method is disclosed, at least comprising the following units (a) to (g) for conducting the steps (1) to (6) of the first method, as shown for example in the composition example in Fig.11.

Outer memory unit (a):

Unit memorizing the amino acid sequence data of a protein or an existing protein data base for use in the step (1) the first method.

Calculation/memory unit (b):

Unit, composed of CPU calculating the frequencies of the occurrences of individual oligopeptides as determined in the step (1), and a memory unit having the memory of the calculation results.

Calculation/memory unit (c):

Unit, composed of CPU calculating the smallest length (n) of oligopeptides as determined in the step (1) and a memory unit having the memory of the length n.

Calculation/memory unit (d):

Unit, composed of CPU calculating the the frequencies of occurrence of each amino acid and the frequencies of occurrence of Aj-oligopeptide and Xi-oligopeptide in the entire proteins as determined in the step (2) and a memory unit having the memory of the calculation results.

Calculation/memory unit (e):

Unit, composed of CPU calculating the Yji value as determined in the step (3) and a memory unit having the memory of the Yji value.

Calculation/memory unit (f):

Unit, composed of CPU calculating the Yj value as determined in the step (4) and a memory unit having the memory of the Yj value.

Calculation/memory unit (g):

Unit, composed of CPU calculating the Zj value as determined in the step (5) and a memory unit having the memory of Zj.

Additionally, the system for predicting a functional site is provided with the following display unit (h) in a preferable embodiment.

Display unit (h):

Unit displaying the Zj value of each amino-acid residue recovered in the calculation/memory unit (g) in a distribution chart.

The system may be equipped with keyboard (i) and control unit (j) and the like as illustrated in Fig.11, in addition to these units (a) to (h).

A further system for predicting a protein functional site is disclosed which is a system for automatically conducting the second method, at least comprising the following units (a) to (i) for carrying out the steps (1) to (9) according to the second method as shown in the composition example in Fig.12.

Outer memory unit (a):

Unit memorizing the amino acid sequence data and an existing protein data base for use in the step (1).

Calculation/memory unit (b):

Unit, composed of CPU calculating the frequencies of the occurrences of individual oligopeptides as determined in the step (1) and a memory unit having the memory of the calculation results.

Calculation/memory unit (c):

Unit, composed of CPU calculating the frequencies of occurrence of each amino acid and the individual frequencies of the occurrences of Aj-oligopeptide and Xi-oligopeptide in the entire proteins as determined in the step (2') and a memory unit having the memory of the calculation results.

Calculation/memory unit (d):

Unit, composed of CPU calculating Yji as determined in the step (3) and a memory unit having the memory of the Yji value.

Calculation/memory unit (e):

Unit, composed of CPU calculating the Y(j, n) value as determined in the step (4) and a memory unit having the memory of the Y(j, n) value.

Calculation/memory unit (f):

Unit, composed of CPU calculating the Z(j, n) value as determined in the steps (5) and (6) and a memory unit having the memory of the Z(j, n) value.

Calculation/memory unit (g):

Unit, composed of CPU calculating the g value as determined in the step (7) and a memory unit having the memory of the g value.

Calculation/memory unit (h):

Unit, composed of CPU calculating the D(j, n) value as determined in the step (8) and a memory unit having the memory of the D(j, n) value.

Calculation/memory unit (i):

Unit, composed of CPU calculating the Wj value as determined in the step (9) and a memory unit having the memory of the Wj value.

Additionally, this system for predicting a functional site may be equipped with an appropriate combination of the following units (j) to (1).

Display unit (j):

Unit displaying the Wj value of each amino-acid residue as recovered with the unit (i) in a distribution chart.

Calculation/memory unit (k):

Unit memorizing an existing database of protein three-dimensional structures or unit preparing a three-dimensional structure model based on an amino acid sequence according to a known method and memorizing the three-dimensional structure.

Display unit (1):

Unit displaying the Wj value of each amino-acid residue in a distribution chart on the three-dimensional structure stored in the database or three-dimensional structure model recorded on the unit (k).

The system may satisfactorily be equipped with keyboard (m) and control unit (n) and the like as illustrated in Fig.12, in addition to these units (a) to (1).

A third method is disclosed which is a method for modifying the function of protein "A", which function has been known, derived from the entire putative proteins of organism "a" with a known genome data or cDNA analysis data, essentially comprising the following steps (1) to (10).

Step (1) :

Extracting proteins closely related to the protein "A" from an existing protein data base and subjecting the proteins to alignment.

Steps (2) to (7):

Subjecting the amino acid sequences of the entire proteins of the organism "a" to the steps (1) to (6) according to first method.

Step (8) :

Selecting at least one amino-acid residue to be subjected to mutation from the amino acid sequence of length (L) of the protein "A" on the basis of the alignment data recovered in the step (1), sequentially repeating the steps (3) to (6) for a variant amino-acid residue of various mutated amino acid sequences where the selected amino-acid residue has been mutated into another amino-acid residue, to determine the Zj value of the variant amino-acid residue.

Step (9):

Selecting a mutated amino acid sequence wherein the Zj value of the variant amino-acid residue as determined in the step (8) is larger or smaller than the Zj value of the intact amino-acid residue as determined in the step (7).

Step (10) :

Preparing a modified gene of the protein "A", which gene encodes the mutant amino acid sequence selected in the step (9), and expressing the modified gene in an appropriate host-vector system to prepare modified protein "A".

A fourth method is disclosed which is a method for modifying the function of protein "B" derived from organism "b" with an unknown genome data or cDNA analysis data, essentially comprising the following steps (1) to (10).

Step (1):

Extracting protein "A" most closely related to protein "B" from the entire putative proteins of organism "a" with a known genome data or cDNA analysis data and subjecting the protein "A" to alignment, or extracting proteins closely related to protein "B" from an existing protein data base to subject the proteins to alignment.

Steps (2) to (8):

Conducting the steps (2) to (8) of the third method over the amino acid sequences of the entire proteins of the organism "a".

Step (9):

Selecting a position that should be mutated and an amino acid residue for which should be substituted wherein the Zj value of the substitued amino-acid residue as determined in the step (8) is larger or smaller than the Zj value of the intact amino-acid residue as determined in the step (7).

Step (10):

Preparing a modified gene of the protein "B" according to a known method, which gene encodes the amino acid sequence mutated at the position to another amino acid residue as selected in the step (9), and expressing the modified gene in an appropriate host-vector system to prepare modified protein "B".

As has been described above, the third and fourth methods for modifying the protein function, comprising the first method for predicting a function site, are characterized in that an unknown functional site of protein is newly found and is subjected to mutation.

Furthermore, the fifth method disclosed herein which is a method for modifying a protein function also utilizes the second method for predicting the function, whereby the method can be carried out in the same manner as in the case of the third method.

The thermophilic DNA polymerase to which the present invention relates is more specifically an enzyme prepared by modifying, in accordance with the fourth method, a thermophilic *Pfu* DNA polymerase derived from *Pyrococcus furiosus* in a genetic engineering manner by the known method for preparing mutant gene (Strategies, Vol.9, p.3-4, 1996) (the thermophilic DNA polymerase of the present invention is sometimes referred to as "modified *Pfu* DNA polymerase").The enzyme can be prepared as follows.

Since the nucleotide sequence of the gene of *Pfu* DNA polymerase is known (Nucleic Acids Research, Vol.21, p.259-265, 1993), the gene of *Pfu* DNA polymerase is prepared by PCR comprising synthetically preparing an oligopeptide complementary to both the ends by using the genome DNA of the *archaebacterium* as template and using the oligopeptide as primer. The DNA fragment of the gene is cloned into a vector, and the gene is subsequently subjected to mutation by the method described in the reference mentioned above. In accordance with the present invention, in particular, the mutation of the gene was executed by nucleotide substitution, so that a part of the amino acid sequence of *Pfu* DNA polymerase might be substituted with the amino-acid residues from *KOD* DNA polymerase. In terms of amino acid sequence, *Pfu* DNA polymerase has about 80 % homology with *KOD* DNA polymerase, and therefore, similar synthetic termination occurs during PCR (Fig.13), but the elongation rate with *KOD* DNA polymerase is about 6-fold the rate with *Pfu* DNApolymerase. By substituting some amino-acid residues of *Pfu* DNA polymerase with some amino-acid residues of *KOD* DNA polymerase, the synthetic termination of the chain elongation might be improved or the elongation rate turns rapid, which possibly enables the recovery of an enzyme capable of elongating a DNA chain under way of synthesis more longer. By expressing in *Escherichia coli* the mutant gene that was mutated in such a manner and recovering and purifying the expression product, the modified Pfu DNA polymerase of the present invention was recovered.

The thermophilic DNA polymerase (modified Pfu DNA polymerase I) of the invention is more specifically an enzyme of the amino acid sequence of SQ ID No.1. The amino acid sequence is a novel sequence prepared by identifying potentially function-modifiable amino-acid residues of the amino acid sequence of the conventionally known Pfu DNA polymerase, according to the method for predicting a functional site, and substituting the amino-acid residues as shown in Table 1. By using the novel enzyme then for DNA synthesis by PCR, for example, the synthetic termination occurring when using the conventional DNA polymerases is almost totally overcome, as shown in the following examples. It is needless to say that template DNA chains to be highly efficiently amplified with the conventional polymerase can be amplified at high efficiency in the same manner.

Furthermore, the thermophilic DNA polymerases (modified *Pfu* DNA polymerases II and III) of the invention are more specifically enzymes of amino acid sequences of SQ ID Nos.6 and 7, which are novel sequences prepared by identifying potentially function-modifiable amino-acid residues of the amino acid sequence of the *Pfu* DNA polymerase, according to the inventive method for predicting a functional site, and substituting the amino-acid residues as shown in Table 1. By using the novel enzymes then for DNA synthesis by PCR, for example, synthetic polymeric products can be recovered at large scales, as shown in the following examples.

Table 1

| Modified DNA polymerases | Positions | Wild-type amino acid | Modified amino acid |
|---|---|---|---|
| I | 2 | Ile | Val |
| | 533 | Phe | Tyr |
| | 538 | Leu | Ile |
| | 540 | Ile | Ser |
| | 545 | Leu | Phe |
| | 546 | Tyr | Phe |
| II | 2 | Ile | Val |
| | 710 | Pro | Arg |
| | 712 | Ser | Arg |
| | 713 | Asn | Asp |
| | 717 | Leu | Pro |
| III | 2 | Ile | Val |
| | 717 | Leu | Pro |

The DNA sequences encoding these modified *Pfu* DNA polymerases include for example the mutant genes of the *Pfu* DNA polymerase gene, as recovered during the process of enzyme preparation. As to these mutant genes, the DNA sequences encoding the amino acid sequences of SQ ID Nos . 1, 6 and 7 for example have been cloned in recombinant plasmids p320, pDP5b17 and pDP5C4, respectively, and these recombinant plasmids have been integrated in *Escherichia coli* HMS174 (DE3) and deposited at the National Institute of Bioscience and Human-Technology, the Agency of Industrial Science and Technology, Japan (Deposit Nos. FERM P-16052, FERM BP-6189 and FERM BP-6190, respectively).

Additionally, the DNA sequences of the present invention may appropriately be designed as DNA sequences with conjugated nucleotide codons corresponding to the individual amino acid residues of SQ ID No.1, 6 or 7.

The thermotolerant DNA polymerases of the present invention may be expressed in microorganisms such as Escherichia coli, which may thereafter be recovered. By inserting and integrating the DNA sequence into an expression vector with an origin replicable in a microorganism, a promoter, a ribosome-binding site, a cDNA cloning site, and a terminator and the like to prepare an expression vector, transforming a host cell with the expression vector and thereafter culturing the resulting transformant, an enzyme encoded by the DNA, sequence can be generated in the microorganism at a large scale.

EXAMPLES

The present invention will now be described more specifically in more detail with reference to examples, but the invention is not limited to the following examples.

Example 1

According to the first method and based on the genome data of Methanococcus jannaschii (Bult et al., Science 273, 1058-1073, 1996), Zj = -logYj (f = -log) was calculated, concerning each amino acid residue in the amino acid sequence (from the N terminus to the C terminus) of a DNA polymerase speculated from the microbial gene MJ0885 which is thought to encode the α-type DNA polymerase. The results are plotted in a distribution chart in Fig.14.

Among the motifs known as the functional sites of the α-type DNA polymerase, furthermore, motif A and motif C were extracted, and the Zj values of the individual amino acid residues were plotted in Fig.15. Fig.15 and Fig.16 below suggest that the Zj = -logYj values of amino acid residue responsible for the function are larger than those of the remaining amino acid residues.

Fig. 16 depicts a distribution chart of the frequency of the value Zj = -logYj for the amino acid sequence of the α-type DNApolymerase encoded byMJ0885. It is confirmed in the figure that amino acid residues with value Zj = -logYj of 4.8 or more are highly possibly amino acid residues responsible for the protein function.

Example 2

Following the chart on Fig.15 in Example 1, the characteristic properties of α-type DNA polymerase Pfu (DDBJ Accession No. D12983) which is derived from Pyrococcus furious were modified, on the basis of the amino acid sequence of an α-type DNA polymerase KOD (DDBJ Accession No. D29671) which is derived from Pyrococcus sp., and the genome data of Methanococcus jannaschii (Bult et al., Science 273, 1058-1073, 1996), and the amino acid sequence of the α-type DNApolymerase (named here as MJ) encoded by MJ0885.

Fig.17 depicts alignment charts of the amino acid sequences of the motif Cs of Pfu, KOD andMJ, with no difference between the region 531 to 544 from Pfu and MJ.

Fig.18 depicts the results of the prediction of functional sites in the amino acid sequences of the motif Cs of Pfu, KOD and MJ according to the method, on the basis of the genome data of Methanococcus jannaschii. The results indicate that mutations Ile540Ser, Leu545Phe, Tyr546Phe, and Ile568Thr increase the Zj = -logYj values of the amino acid residues. Furthermore, the values Zj = -logYj of Asp541 and Ala547 are increased. By subjecting the α-type DNApolymerase MJ of Methanococcus jannaschii to such mutation, the resulting sequence turns more unique (specific) which possibly brings about the improvement of some function.

Example 3

Based on the genome data of Methanococcus jannaschii (Bult et al., Science 273, 1058-1073, 1996), the values Z (j, 1) = -logY(j, 1), Z (j, 3) = -logY(j, 3), Z (j, 4) = -logY(j, 4), and Z (j, 5) = -logY(j, 5) were calculated, concerning the individual amino acid residues of the amino acid sequence (from the N to C termini) of a DNA polymerase speculated on the basis of the microbial gene MJ0885 which is believed to encode the α-type DNA polymerase, according to the second method, so that Wj = Z (j, 3) - Z(j, 1) (h = Z(j, 3) - Z(j, 1)) was calculated. Similarly, Wj = Z (j, 4) - Z(j, 3) (h = Z(j, 4) - Z(j, 3)) and Wj = Z(j, 5) - Z(j, 3) (h = Z(j, 5) - Z(j, 3)) were also calculated.

Fig. 19 depicts the results of the 100 residues from the N terminus in a plotted distribution chart. Given h = Z (j, 5) - Z(j, 3), regions with significantly different distributions from those of the remaining two cases are present in the region from the 35-th to 60-th residues and the like. The distributions indicate that smaller Wj = Z (j, 5) - Z(j, 3) more specifically characterizes the amino acid sequence.

Among the motifs known as the functional sites of the α-type DNApolymerases, furthermore, regions containing exoI, exoII, motif A, motif B and motif C were extracted, and subsequently, the Wj values of the individual amino acid residues are plotted in Fig. 20. As shown in Fig. 20, the regions with the characteristic reduction of Wj are consistent with the functional sites.

Example 4

Fig.21 depicts the values Wj = D(j, 3) and Wj = D(j, 5) of individual amino acid residues in the regions containing the exoI, exoII, motif A, motif B and motif C extracted among the motifs known as the functional sites in the α-type DNA polymerases (h=D(j, 3) and h = D(j, 5)). Amino acid residues with Wj = D(j, n) of 2 or more or of 2 or less are present outside the motifs, and these amino acid residues are candidates of new functional sites.

Example 5

Fig.22 depicts in a dark color the positions of amino acid residues having Wj = D (j, 3) of 2 or more or of 2 or less in the amino acid sequence of MJ0232, which is speculated as enolase of Methanococcus jannaschii, on a three-dimensional structure model prepared on the basis of the enolase of budding yeast. It is indicated that residues positioned apart on the amino acid sequence are closely positioned on the three-dimensional structure.

Example 6

Modified Pfu DNA polymerase I was prepared, by applying the mutation of putative amino acid residues for improving the function of the DNA polymerase MJ in Example 2 to the Pfu DNA polymerase.

(1) Preparation of modified Pfu DNA polymerase gene Cloning of Pfu DNA polymerase gene:

Following the nucleotide sequence of Pfu DNA polymerase gene (Nucleic Acids Research, Vol.21, p.259-265, 1993), a PCR primer was prepared, for amplifying the objective gene by PCR by using the genome DNA of P. furiosus as template, which was then cloned in an expression vector for Escherichia coli. The detail is described below.

P. furiosus DSM3638 was cultured according to the method described in the reference described above. First, the culture medium described in the reference was prepared, followed by sterilization at a high temperature underpressure, and subsequently, nitrogen gas was purged into the resulting culture medium. The bacterium was inoculated into the culture medium, for stationary culturing at 95°C for 15 hours. From 200 ml of the culture broth were recovered the bacteria of about 0.5 mg by centrifugation. The collected bacteria were suspended in buffer A (10 mM Tris/HCl (pH 8.0), .1 mM EDTA, 100 mM NaCl), followed by addition of 1 ml of 10 % SDS and subsequent agitation, and to the resulting suspension was added 0.5 mg of proteinase K for reaction at 55 °C for 60 minutes. The reaction solution was extracted sequentially in phenol, phenol/chloroform, and chloroform, and to the extract was added ethanol to make the DNA insoluble, which was then recovered. The resulting DNA was dissolved in 1 ml of TE buffer (10 mM Tris/HCl (pH 8.0), 1 mM EDTA), followed by addition of 0. 5 mg RNase A for reaction at 37°C for 60 minutes and re-extraction sequentially in phenol, phenol/chloroform, and chloroform, and subsequent ethanol precipitation, to recover the DNA, which was then dissolved in the TE buffer, to recover the DNA at about 0.3 mg.

For PCR amplification of the objective DNA polymerase gene, then, two primer DNAs of SQ ID Nos.2 and 3 were synthesized on the basis of the known sequence data. More specifically, it was designed that the initiation codon ATG of the objective gene and a restriction nuclease NcoI sequence (5'-CCATGG-3') might be introduced in the forward primer sequence, while the reverse primer might be conjugated at an appropriate position downstream the termination codon. PCR was conducted in a reaction system of 50 μl, by using 2 μg of P. furiosus DNA and 10 pmol each of the primers under conditions for LATaq (manufactured by TaKaRa Brewery) and attached buffers. The cycle conditions were as follows; 93°C/3 minutes prior to the addition of the enzyme, and 30 cycles of each cycle composed of 94°C for 0.5 minute, 55°C for 0.5 minute and 72°C for 1.0 minute. The amplified DNA fragment was purified, followed by treatment with NcoI, and the resulting DNA fragment was similarly cleaved with NcoI and subsequently blunt ended, and the resulting fragment was then integrated downstream the T7 promote of an NcoI-treated expression vector pET15-b. The expression vector was defined as pDPWT100, to confirm the nucleotide sequence of the inserted gene. Modification of Pfu DNA polymerase gene:

According to the known method (Strategies, Vol.9, p. 3-4, 1996) and for the expression vector pDPWT100 with the cloned Pfu DNA polymerase gene integrated therein, a modified Pfu DNA polymerase gene was prepared on the expression vector pDPWT100 by using oligopeptides containing desired mutations (SQ ID Nos. 4 and 5) and the mutation induction kit manufactured by Promega Corporation, whereby an expression vector pDP320 was constructed. By determining the nucleotide sequence of the modified gene, furthermore, the amino acid sequence of the modified Pfu DNA polymerase (SQ ID No.1) was verified.

(2) Expression and purification of the modified Pfu DNA polymerase in Escherichia coli

The gene of the modified Pfu DNApolymerase I was expressed in Escherichia coli as follows, which was then purified.

The expression vector pDP320 with the modified Pfu DNA polymerase gene was inserted in Escherichia coli HMS174 (DE3) and cultured in an LB culture medium supplemented with IPTG to a final concentration of 0.1 mM for 14 hours, to induce the expression of the enzyme in the bacteria of the Escherichia coli. After recovering the bacteria by centrifugation, a modified Pfu DNA polymerase was extracted under ultrasonic treatment in a buffer containing 150 mM Tris/HCl (pH 7.5), 2 mM EDTA, 0.24 mM APMSF and 0.2 % Tween 20. The crude extract solution was thermally treated at 80°C for 15 minutes, to inactivate the DNA polymerase derived from Escherichia coli and partially purify the DNA polymerase of the present invention. The partially purified fraction was dialyzed against a buffer composed of 50 mM Tris/HCl(pH 7.5), 1 mM EDTA, 0.2 % Tween 20, 7 mM 2-mercaptoethanol and 10 % glycerol. At the stage was detected a DNA polymerizing activity specific to the modified Pfu DNA polymerase I.

Example 7

By using the modified Pfu DNA polymerase I partially purified in Example 6, the primer elongation reaction of a DNA chain complimentary to the template DNA was tested.

One μg of the partially purified enzyme fraction described above was placed in 20 μl of a reaction solution containing 20 m Tris/HCl (pH 8.0), 2 mM $MgCl_2$, 50 μg/ml BSA, 0.1 % Triton X-100, 1 mM each of cold dNTPs (0.1 mM for dCTP), 0. 63 μg of pBLUESCRIPT plasmid prepared by annealing together 10 μ Ci of [α-$^{32}$P] DCTP and a primer of M13(-21), for reaction at 75°C for one minute and 3 minutes. The elongated DNA chain was separated by electrophoresis on a polyacrylamide gel containing 8M urea, and the resulting pattern was analyzed with an image analyzer. As a control, additionally, the conventional wild-type Pfu DNA polymerase was used for the same DNA synthesis.

The results are shown in Fig 23. When the conventional wild-type Pfu DNA polymerase was used, at least 10 bands indicating the presence of incomplete DNA fragments due to synthetic termination were observed. However, these bands disappeared during the DNA synthesis with the modified Pfu DNA polymerase I of the present invention. Alternatively, no difference in the accumulation of highly elongated DNA fragments around 1000 bases was observed.

Example 8

Pfu DNA polymerases II and III of this invention were prepared.

(1) Preparation of modified Pfu DNA polymerase gene

In the same manner as in Example 6(1), the Pfu DNA polymerase gene was cloned, to prepare modified genes II and III as follows.

Preparation of modified Pfu DNA polymerase II:

According to the known method (Strategies, Vol.9, p.3-4, 1996) and for the expression vector pDPWT100 with the cloned Pfu DNApolymerase gene integrated therein, the gene of modified Pfu DNA polymerase II was prepared on the expression vector pDPWT100 by using oligopeptides containing desired mutations (SQ ID Nos.8 and 9) and the mutation induction kit manufactured by Promega Corporation, whereby an expression vector pDP5b17 was constructed. By determining the nucleotide sequence of the modified gene, furthermore, the amino acid sequence of the modified Pfu DNA polymerase II (SQ ID No.6) was confirmed. Preparation of the gene of modified Pfu DNA polymerase III:

By the same method as described above except for the use of the oligonucleotides of SQ ID Nos.10 and 11, the gene of modified Pfu DNA polymerase III was prepared, to construct an expression vector pDP5C4. By determining the nucleotide sequence of the modified gene, the amino acid sequence (SQ ID No.7) of the modified Pfu DNA polymerase III was confirmed.

(2) Expression in Escherichia coli and purification of the modified Pfu DNA polymerases II and III.

The genes of the modified Pfu DNA polymerases II and III, thus prepared, were expressed in Escherichia coli as follows, which were then purified.

The expression vectors pDP5b17 and pDP5C4 were independently inserted in Escherichia coli HMS174 (DE3) and cultured in an LB culture medium supplemented with IPTG to a final concentration of 0.1 mM for 14 hours, to induce the expression of the enzymes in the Escherichia coli. After recovering the bacteria by centrifugation, modified Pfu DNA polymerases II and III were extracted, with ultrasonic treatment, in a buffer containing 150 mM Tris/HCl(pH 7.5), 2 mM EDTA, 0.24 mM APMSF and 0.2 % Tween 20. The crude extract solution was thermally treated at 80°C for 15 minutes, to inactivate the DNA polymerases derived from Escherichia coli and partially purify the modified DNA polymerases II and III. The partially purified fractions were dialyzed against a buffer composed of 50 mM Tris/HCl(pH 7.5), 1 mM EDTA, 0.2 % Tween 20, 7 mM 2-mercaptoethanol and 10 % glycerol. At the stage were detected DNA polymerizing activities specific to the modified Pfu DNA polymerases II and III.

Example 9

By using the modified Pfu DNA polymerases II and III partiallypurified in Example 8, the primer elongation reaction of a DNA chain complimentary to the template DNA was tested.

One μg of each of the partially purified enzyme fractions described above was placed in 20 μl of a reaction solution containing 20 mM Tris/HCl (pH 8.0), 2 mM $MgCl_2$, 50 μg/ml BSA, 0.1% Triton X-100, 1 mM each of cold dNTPs (0.1 mM for dCTP), 0.63 μg of pBLUESCRIPT plasmid prepared by annealing together 10 μCi of [α-$^{32}$P]dCTP and a

primer M13 (-21), for reaction at 75 °C for one minute and 3 minutes. The elongated DNA chain was separated by electrophoresis on a polyacrylamide gel containing 8M urea, and the resulting pattern was analyzed with an image analyzer. As a control, additionally, the conventional wild-type Pfu DNA polymerase was used for the same DNA synthesis.

The results are shown in Fig.24. When the conventional wild-type Pfu DNA polymerase was used, bands indicating the presence of incomplete DNA fragments were observed, because of the presence of a large region at about 1000 bases where synthetic termination occurred. However, the yield of synthesized products including those of bands of about 1000 bases was elevated during the DNA synthesis with the modified Pfu DNA polymerases II and III of the present invention, together with bands indicating the presence of more polymeric (more elongated) PCR products under observation.

The results described above indicate that the DNA polymerases of the present invention canmore markedly elongate DNA fragments in the course of synthesis during the DNA synthesis by PCR.

INDUSTRIAL APPLICABILITY

As described herein, the functional site of a protein with no information of function which obtained by genome analysis or cDNA analysis can be predicted. A novel functional site of a protein with a known function can also be predicted.

The thermophilic DNA polymerases provided by the present invention can highly efficiently synthesize and amplify the full length of a DNA fragment by PCR, whereby the in vitro synthesis andamplificationof a DNA fragment and the nucleotide sequencing thereof can be attained at a high precision in a simple manner.

SEQUENCE LISTING

SEQ ID NO.: 1
LENGTH: 775
TYPE: amino acid
MOLECULE TYPE: protein
SEQUENCE

Met Val Leu Asp Val Asp Tyr Ile Thr Glu Glu Gly Lys Pro Val Ile
1 5 10 15

Arg Leu Phe Lys Lys Glu Asn Gly Lys Phe Lys Ile Glu His Asp Arg
20 25 30

Thr Phe Arg Pro Tyr Ile Tyr Ala Leu Leu Arg Asp Asp Ser Lys Ile
35 40 45

Glu Glu Val Lys Lys Ile Thr Gly Glu Arg His Gly Lys Ile Val Arg
50 55 60

Ile Val Asp Val Glu Lys Val Glu Lys Lys Phe Leu Gly Lys Pro Ile
65 70 75 80

Thr Val Trp Lys Leu Tyr Leu Glu His Pro Gln Asp Val Pro Thr Ile
85 90 95

Arg Glu Lys Val Arg Glu His Pro Ala Val Val Asp Ile Phe Glu Tyr
100 105 110

Asp Ile Pro Phe Ala Lys Arg Tyr Leu Ile Asp Lys Gly Leu Ile Pro
115 120 125

Met Glu Gly Glu Glu Glu Leu Lys Ile Leu Ala Phe Asp Ile Glu Thr
130 135 140

Leu Tyr His Glu Gly Glu Glu Phe Gly Lys Gly Pro Ile Ile Met Ile
145 150 155 160
Ser Tyr Ala Asp Glu Asn Glu Ala Lys Val Ile Thr Trp Lys Asn Ile
165 170 175
Asp Leu Pro Tyr Val Glu Val Val Ser Ser Glu Arg Glu Met Ile Lys
180 185 190
Arg Phe Leu Arg Ile Ile Arg Glu Lys Asp Pro Asp Ile Ile Val Thr
195 200 205
Tyr Asn Gly Asp Ser Phe Asp Phe Pro Tyr Leu Ala Lys Arg Ala Glu
210 215 220
Lys Leu Gly Ile Lys Leu Thr Ile Gly Arg Asp Gly Ser Glu Pro Lys
225 230 235 240
Met Gln Arg Ile Gly Asp Met Thr Ala Val Glu Val Lys Gly Arg Ile
245 250 255
His Phe Asp Leu Tyr His Val Ile Thr Arg Thr Ile Asn Leu Pro Thr
260 265 270
Tyr Thr Leu Glu Ala Val Tyr Glu Ala Ile Phe Gly Lys Pro Lys Glu
275 280 285
Lys Val Tyr Ala Asp Glu Ile Ala Lys Ala Trp Glu Ser Gly Glu Asn
290 295 300
Leu Glu Arg Val Ala Lys Tyr Ser Met Glu Asp Ala Lys Ala Thr Tyr
305 310 315 320
Glu Leu Gly Lys Glu Phe Leu Pro Met Glu Ile Gln Leu Ser Arg Leu
325 330 335

Val Gly Gln Pro Leu Trp Asp Val Ser Arg Ser Ser Thr Gly Asn Leu
340 345 350

Val Glu Trp Phe Leu Leu Arg Lys Ala Tyr Glu Arg Asn Glu Val Ala
355 360 365

Pro Asn Lys Pro Ser Glu Glu Glu Tyr Gln Arg Arg Leu Arg Glu Ser
370 375 380

Tyr Thr Gly Gly Phe Val Lys Glu Pro Glu Lys Gly Leu Trp Glu Asn
385 390 395 400

Ile Val Tyr Leu Asp Phe Arg Ala Leu Tyr Pro Ser Ile Ile Ile Thr
405 410 415

His Asn Val Ser Pro Asp Thr Leu Asn Leu Glu Gly Cys Lys Asn Tyr
420 425 430

Asp Ile Ala Pro Gln Val Gly His Lys Phe Cys Lys Asp Ile Pro Gly
435 440 445

Phe Ile Pro Ser Leu Leu Gly His Leu Leu Glu Glu Arg Gln Lys Ile
450 455 460

Lys Thr Lys Met Lys Glu Thr Gln Asp Pro Ile Glu Lys Ile Leu Leu
465 470 475 480

Asp Tyr Arg Gln Lys Ala Ile Lys Leu Leu Ala Asn Ser Phe Tyr Gly
485 490 495

Tyr Tyr Gly Tyr Ala Lys Ala Arg Trp Tyr Cys Lys Glu Cys Ala Glu
500 505 510

Ser Val Thr Ala Trp Gly Arg Lys Tyr Ile Glu Leu Val Trp Lys Glu
515 520 525

Leu Glu Glu Lys Tyr Gly Phe Lys Val Ile Tyr Ser Asp Thr Asp Gly
530 535 540

Phe Phe Ala Thr Ile Pro Gly Gly Glu Ser Glu Glu Ile Lys Lys Lys
545 550 555 560

Ala Leu Glu Phe Val Lys Tyr Ile Asn Ser Lys Leu Pro Gly Leu Leu
565 570 575

Glu Leu Glu Tyr Glu Gly Phe Tyr Lys Arg Gly Phe Phe Val Thr Lys
580 585 590

Lys Arg Tyr Ala Val Ile Asp Glu Glu Gly Lys Val Ile Thr Arg Gly
595 600 605

Leu Glu Ile Val Arg Arg Asp Trp Ser Glu Ile Ala Lys Glu Thr Gln
610 615 620

Ala Arg Val Leu Glu Thr Ile Leu Lys His Gly Asp Val Glu Glu Ala
625 630 635 640

Val Arg Ile Val Lys Glu Val Ile Gln Lys Leu Ala Asn Tyr Glu Ile
645 650 655

Pro Pro Glu Lys Leu Ala Ile Tyr Glu Gln Ile Thr Arg Pro Leu His
660 665 670

Glu Tyr Lys Ala Ile Gly Pro His Val Ala Val Ala Lys Lys Leu Ala
675 680 685

Ala Lys Gly Val Lys Ile Lys Pro Gly Met Val Ile Gly Tyr Ile Val
690 695 700

Leu Arg Gly Asp Gly Pro Ile Ser Asn Arg Ala Ile Leu Ala Glu Glu
705 710 715 720

Tyr Asp Pro Lys Lys His Lys Tyr Asp Ala Glu Tyr Tyr Ile Glu Asn

725 730 735

Gln Val Leu Pro Ala Val Leu Arg Ile Leu Glu Gly Phe Gly Tyr Arg

740 745 750

Lys Glu Asp Leu Arg Tyr Gln Lys Thr Arg Gln Val Gly Leu Thr Ser

755 760 765

Trp Leu Asn Ile Lys Lys Ser

770 775

SEQ ID NO.: 2
LENGTH: 35
TYPE: nucleic acid
STRANDEDNESS: single
TOPOLOGY: linear
MOLECULE TYPE: synthetic DNA
SEQUENCE
GTGGGGAGCA CCATGGTTTT AGATGTGGAT TACAT 35
SEQ ID NO.: 3
LENGTH: 35
TYPE: nucleic acid
STRANDEDNESS: single
TOPOLOGY: linear
MOLECULE TYPE: synthetic DNA
SEQUENCE
GCATGCAGAT AGACCATTTC TAACGAAGGC GTTTG 35
SEQ ID NO.: 4
LENGTH: 66
TYPE: nucleic acid
STRANDEDNESS: single
TOPOLOGY: linear
MOLECULE TYPE: synthetic DNA
SEQUENCE

CTCGAAGAAA AGTATGGATT TAAAGTCATC TACAGTGACA CTGATGGTTT CTTTGCAACT

ATCCCA

66

SEQ ID NO.: 5
LENGTH: 66
TYPE: nucleic acid
STRANDEDNESS: single
TOPOLOGY: linear

MOLECULE TYPE: synthetic DNA
SEQUENCE

TGGGATAGTT GCAAAGAAAC CATCAGTGTC ACTGTAGATG ACTTTAAATC CATACTTTTC

TTCGAG

66

SEQ ID NO.: 6
LENGTH: 775
TYPE: amino acid
MOLECULE TYPE: protein
SEQUENCE

Met Val Leu Asp Val Asp Tyr Ile Thr Glu Glu Gly Lys Pro Val Ile
1 5 10 15

Arg Leu Phe Lys Lys Glu Asn Gly Lys Phe Lys Ile Glu His Asp Arg
20 25 30

Thr Phe Arg Pro Tyr Ile Tyr Ala Leu Leu Arg Asp Asp Ser Lys Ile
35 40 45

Glu Glu Val Lys Lys Ile Thr Gly Glu Arg His Gly Lys Ile Val Arg
50 55 60

Ile Val Asp Val Glu Lys Val Glu Lys Lys Phe Leu Gly Lys Pro Ile
65 70 75 80

Thr Val Trp Lys Leu Tyr Leu Glu His Pro Gln Asp Val Pro Thr Ile
85 90 95

Arg Glu Lys Val Arg Glu His Pro Ala Val Val Asp Ile Phe Glu Tyr
100 105 110

Asp Ile Pro Phe Ala Lys Arg Tyr Leu Ile Asp Lys Gly Leu Ile Pro
115 120 125

Met Glu Gly Glu Glu Glu Leu Lys Ile Leu Ala Phe Asp Ile Glu Thr
130 135 140

Leu Tyr His Glu Gly Glu Glu Phe Gly Lys Gly Pro Ile Ile Met Ile
145 150 155 160
Ser Tyr Ala Asp Glu Asn Glu Ala Lys Val Ile Thr Trp Lys Asn Ile
165 170 175
Asp Leu Pro Tyr Val Glu Val Val Ser Ser Glu Arg Glu Met Ile Lys
180 185 190
Arg Phe Leu Arg Ile Ile Arg Glu Lys Asp Pro Asp Ile Ile Val Thr
195 200 205
Tyr Asn Gly Asp Ser Phe Asp Phe Pro Tyr Leu Ala Lys Arg Ala Glu
210 215 220
Lys Leu Gly Ile Lys Leu Thr Ile Gly Arg Asp Gly Ser Glu Pro Lys
225 230 235 240
Met Gln Arg Ile Gly Asp Met Thr Ala Val Glu Val Lys Gly Arg Ile
245 250 255
His Phe Asp Leu Tyr His Val Ile Thr Arg Thr Ile Asn Leu Pro Thr
260 265 270
Tyr Thr Leu Glu Ala Val Tyr Glu Ala Ile Phe Gly Lys Pro Lys Glu
275 280 285
Lys Val Tyr Ala Asp Glu Ile Ala Lys Ala Trp Glu Ser Gly Glu Asn
290 295 300
Leu Glu Arg Val Ala Lys Tyr Ser Met Glu Asp Ala Lys Ala Thr Tyr
305 310 315 320
Glu Leu Gly Lys Glu Phe Leu Pro Met Glu Ile Gln Leu Ser Arg Leu
325 330 335

Val Gly Gln Pro Leu Trp Asp Val Ser Arg Ser Ser Thr Gly Asn Leu
340 345 350

Val Glu Trp Phe Leu Leu Arg Lys Ala Tyr Glu Arg Asn Glu Val Ala
355 360 365

Pro Asn Lys Pro Ser Glu Glu Glu Tyr Gln Arg Arg Leu Arg Glu Ser
370 375 380

Tyr Thr Gly Gly Phe Val Lys Glu Pro Glu Lys Gly Leu Trp Glu Asn
385 390 395 400

Ile Val Tyr Leu Asp Phe Arg Ala Leu Tyr Pro Ser Ile Ile Ile Thr
405 410 415

His Asn Val Ser Pro Asp Thr Leu Asn Leu Glu Gly Cys Lys Asn Tyr
420 425 430

Asp Ile Ala Pro Gln Val Gly His Lys Phe Cys Lys Asp Ile Pro Gly
435 440 445

Phe Ile Pro Ser Leu Leu Gly His Leu Leu Glu Glu Arg Gln Lys Ile
450 455 460

Lys Thr Lys Met Lys Glu Thr Gln Asp Pro Ile Glu Lys Ile Leu Leu
465 470 475 480

Asp Tyr Arg Gln Lys Ala Ile Lys Leu Leu Ala Asn Ser Phe Tyr Gly
485 490 495

Tyr Tyr Gly Tyr Ala Lys Ala Arg Trp Tyr Cys Lys Glu Cys Ala Glu
500 505 510

Ser Val Thr Ala Trp Gly Arg Lys Tyr Ile Glu Leu Val Trp Lys Glu
515 520 525

Leu Glu Glu Lys Phe Gly Phe Lys Val Leu Tyr Ile Asp Thr Asp Gly
530 535 540

Leu Tyr Ala Thr Ile Pro Gly Gly Glu Ser Glu Glu Ile Lys Lys Lys
545 550 555 560

Ala Leu Glu Phe Val Lys Tyr Ile Asn Ser Lys Leu Pro Gly Leu Leu
565 570 575

Glu Leu Glu Tyr Glu Gly Phe Tyr Lys Arg Gly Phe Phe Val Thr Lys
580 585 590

Lys Arg Tyr Ala Val Ile Asp Glu Glu Gly Lys Val Ile Thr Arg Gly
595 600 605

Leu Glu Ile Val Arg Arg Asp Trp Ser Glu Ile Ala Lys Glu Thr Gln
610 615 620

Ala Arg Val Leu Glu Thr Ile Leu Lys His Gly Asp Val Glu Glu Ala
625 630 635 640

Val Arg Ile Val Lys Glu Val Ile Gln Lys Leu Ala Asn Tyr Glu Ile
645 650 655

Pro Pro Glu Lys Leu Ala Ile Tyr Glu Gln Ile Thr Arg Pro Leu His
660 665 670

Glu Tyr Lys Ala Ile Gly Pro His Val Ala Val Ala Lys Lys Leu Ala
675 680 685

Ala Lys Gly Val Lys Ile Lys Pro Gly Met Val Ile Gly Tyr Ile Val
690 695 700

Leu Arg Gly Asp Gly Arg Ile Arg Asp Arg Ala Ile Pro Ala Glu Glu
705 710 715 720

Tyr Asp Pro Lys Lys His Lys Tyr Asp Ala Glu Tyr Tyr Ile Glu Asn
725 730 735

Gln Val Leu Pro Ala Val Leu Arg Ile Leu Glu Gly Phe Gly Tyr Arg
740 745 750

Lys Glu Asp Leu Arg Tyr Gln Lys Thr Arg Gln Val Gly Leu Thr Ser
755 760 765

Trp Leu Asn Ile Lys Lys Ser
770 775

SEQ ID NO.: 7
LENGTH: 775
TYPE: amino acid
MOLECULE TYPE: protein
SEQUENCE

Met Val Leu Asp Val Asp Tyr Ile Thr Glu Glu Gly Lys Pro Val Ile
1 5 10 15

Arg Leu Phe Lys Lys Glu Asn Gly Lys Phe Lys Ile Glu His Asp Arg
20 25 30

Thr Phe Arg Pro Tyr Ile Tyr Ala Leu Leu Arg Asp Asp Ser Lys Ile
35 40 45

Glu Glu Val Lys Lys Ile Thr Gly Glu Arg His Gly Lys Ile Val Arg
50 55 60

Ile Val Asp Val Glu Lys Val Glu Lys Lys Phe Leu Gly Lys Pro Ile
65 70 75 80

Thr Val Trp Lys Leu Tyr Leu Glu His Pro Gln Asp Val Pro Thr Ile

85 90 95

Arg Glu Lys Val Arg Glu His Pro Ala Val Val Asp Ile Phe Glu Tyr

100 105 110

Asp Ile Pro Phe Ala Lys Arg Tyr Leu Ile Asp Lys Gly Leu Ile Pro

115 120 125

Met Glu Gly Glu Glu Glu Leu Lys Ile Leu Ala Phe Asp Ile Glu Thr

130 135 140

Leu Tyr His Glu Gly Glu Glu Phe Gly Lys Gly Pro Ile Ile Met Ile

145 150 155 160

Ser Tyr Ala Asp Glu Asn Glu Ala Lys Val Ile Thr Trp Lys Asn Ile

165 170 175

Asp Leu Pro Tyr Val Glu Val Val Ser Ser Glu Arg Glu Met Ile Lys

180 185 190

Arg Phe Leu Arg Ile Ile Arg Glu Lys Asp Pro Asp Ile Ile Val Thr

195 200 205

Tyr Asn Gly Asp Ser Phe Asp Phe Pro Tyr Leu Ala Lys Arg Ala Glu

210 215 220

Lys Leu Gly Ile Lys Leu Thr Ile Gly Arg Asp Gly Ser Glu Pro Lys

225 230 235 240

Met Gln Arg Ile Gly Asp Met Thr Ala Val Glu Val Lys Gly Arg Ile

245 250 255

His Phe Asp Leu Tyr His Val Ile Thr Arg Thr Ile Asn Leu Pro Thr

260 265 270

Tyr Thr Leu Glu Ala Val Tyr Glu Ala Ile Phe Gly Lys Pro Lys Glu
275 280 285

Lys Val Tyr Ala Asp Glu Ile Ala Lys Ala Trp Glu Ser Gly Glu Asn
290 295 300

Leu Glu Arg Val Ala Lys Tyr Ser Met Glu Asp Ala Lys Ala Thr Tyr
305 310 315 320

Glu Leu Gly Lys Glu Phe Leu Pro Met Glu Ile Gln Leu Ser Arg Leu
325 330 335

Val Gly Gln Pro Leu Trp Asp Val Ser Arg Ser Ser Thr Gly Asn Leu
340 345 350

Val Glu Trp Phe Leu Leu Arg Lys Ala Tyr Glu Arg Asn Glu Val Ala
355 360 365

Pro Asn Lys Pro Ser Glu Glu Glu Tyr Gln Arg Arg Leu Arg Glu Ser
370 375 380

Tyr Thr Gly Gly Phe Val Lys Glu Pro Glu Lys Gly Leu Trp Glu Asn
385 390 395 400

Ile Val Tyr Leu Asp Phe Arg Ala Leu Tyr Pro Ser Ile Ile Ile Thr
405 410 415

His Asn Val Ser Pro Asp Thr Leu Asn Leu Glu Gly Cys Lys Asn Tyr
420 425 430

Asp Ile Ala Pro Gln Val Gly His Lys Phe Cys Lys Asp Ile Pro Gly
435 440 445

Phe Ile Pro Ser Leu Leu Gly His Leu Leu Glu Glu Arg Gln Lys Ile
450 455 460

Lys Thr Lys Met Lys Glu Thr Gln Asp Pro Ile Glu Lys Ile Leu Leu
465 470 475 480

Asp Tyr Arg Gln Lys Ala Ile Lys Leu Leu Ala Asn Ser Phe Tyr Gly
485 490 495

Tyr Tyr Gly Tyr Ala Lys Ala Arg Trp Tyr Cys Lys Glu Cys Ala Glu
500 505 510

Ser Val Thr Ala Trp Gly Arg Lys Tyr Ile Glu Leu Val Trp Lys Glu
515 520 525

Leu Glu Glu Lys Phe Gly Phe Lys Val Leu Tyr Ile Asp Thr Asp Gly
530 535 540

Leu Tyr Ala Thr Ile Pro Gly Gly Glu Ser Glu Glu Ile Lys Lys Lys
545 550 555 560

Ala Leu Glu Phe Val Lys Tyr Ile Asn Ser Lys Leu Pro Gly Leu Leu
565 570 575

Glu Leu Glu Tyr Glu Gly Phe Tyr Lys Arg Gly Phe Phe Val Thr Lys
580 585 590

Lys Arg Tyr Ala Val Ile Asp Glu Glu Gly Lys Val Ile Thr Arg Gly
595 600 605

Leu Glu Ile Val Arg Arg Asp Trp Ser Glu Ile Ala Lys Glu Thr Gln
610 615 620

Ala Arg Val Leu Glu Thr Ile Leu Lys His Gly Asp Val Glu Glu Ala
625 630 635 640

Val Arg Ile Val Lys Glu Val Ile Gln Lys Leu Ala Asn Tyr Glu Ile
645 650 655

Pro Pro Glu Lys Leu Ala Ile Tyr Glu Gln Ile Thr Arg Pro Leu His
660 665 670

Glu Tyr Lys Ala Ile Gly Pro His Val Ala Val Ala Lys Lys Leu Ala
675 680 685

Ala Lys Gly Val Lys Ile Lys Pro Gly Met Val Ile Gly Tyr Ile Val
690 695 700

Leu Arg Gly Asp Gly Pro Ile Ser Asn Arg Ala Ile Pro Ala Glu Glu
705 710 715 720

Tyr Asp Pro Lys Lys His Lys Tyr Asp Ala Glu Tyr Tyr Ile Glu Asn
725 730 735

Gln Val Leu Pro Ala Val Leu Arg Ile Leu Glu Gly Phe Gly Tyr Arg
740 745 750

Lys Glu Asp Leu Arg Tyr Gln Lys Thr Arg Gln Val Gly Leu Thr Ser
755 760 765

Trp Leu Asn Ile Lys Lys Ser
770 775

SEQ ID NO.: 8
LENGTH: 49
TYPE: nucleic acid
STRANDEDNESS: single
TOPOLOGY: linear
MOLECULE TYPE: synthetic DNA
SEQUENCE
AGAGGCGATG GTCGAATTCG CGATAGGGCA ATTCCAGCTG AGGAATACG 49
SEQ ID NO.: 9
LENGTH: 49
TYPE: nucleic acid
STRANDEDNESS: single
TOPOLOGY: linear
MOLECULE TYPE: synthetic DNA
SEQUENCE
CGTATTCCTC AGCTGGAATT GCCCTATCGC GAATTCGACC ATCGCCTCT 49
SEQ ID NO.: 10
LENGTH: 40
TYPE: nucleic acid
STRANDEDNESS: single

TOPOLOGY: linear
MOLECULE TYPE: synthetic DNA
SEQUENCE
CCAATTAGCA ATAGCCCAAT TCCAGCTGAG GAATACGATC 40
SEQ ID NO.: 11
LENGTH: 40
TYPE: nucleic acid
STRANDEDNESS: single
TOPOLOGY: linear
MOLECULE TYPE: synthetic DNA
SEQUENCE
GATCGTATTC CTCAGCTGGA ATTGCCCTAT TGCTAATTGG 40

**Claims**

1. A thermophilic DNA polymerase, prepared by artificially modifying the amino acid sequence of Pfu DNA polymerase so that either

   (a) the elongation of synthesized DNA chain might not be terminated intermediately during the catalysis for the synthesis of a DNA chain complimentary to a single-stranded DNA, or
   (b) the synthesized DNA chain might be more elongated during the catalysis for the synthesis of a DNA chain complimentary to a single-stranded DNA, and

   wherein the thermophilic DNA polymerase of part (a), comprises the amino acid sequence of SEQ ID No. 1, and the DNA polymerase of part (b), comprises the amino acid sequence of SEQ ID No. 6 or SEQ ID No. 7.

2. A DNA sequence encoding the amino acid sequence of SEQ ID No. 1, SEQ ID No. 6 or SEQ ID No. 7.

3. A recombinant vector carrying the DNA sequence of claim 2.

4. The recombinant vector according to claim 3, which is a recombinant plasmid pDP 5b17 carried on *Escherichia coli* HMS 174 (DE3) /pDP 5b17 deposited at the National Institute of Bioscience and Human Technology, the Agency of Industrial Science and Technology, Japan under Accession No. FERM BP-6189 for SQ ID No. 6 or a recombinant plasmid pDP 5c4 carried on Escherichia coli HMS 174 (DE3)/ pDP 5c4 deposited at the National Institute of Bioscience and Human Technology, the Agency of Industrial Science and Technology, Japan under Accession No. FERM BP-6190 for SEQ ID No. 7.

5. A method for preparing a DNA polymerase, comprising culturing a cell transformed with an expression vector carrying the DNA sequence of claim 2 and isolating and purifying the objective enzyme produced in the culture medium.

**Patentansprüche**

1. Eine thermophile DNA-Polymerase, hergestellt durch künstliche Modifizierung der Aminosäuresequenz von Pfu-DNA-Polymerase, so dass entweder

   (a) die Verlängerung der synthetisierten DNA-Kette eventuell nicht intermediär während der Katalyse zur Synthese einer DNA-Kette, die komplementär zu einer einzelsträngigen DNA ist, beendet wird, oder
   (b) die synthetisierte DNA-Kette eventuell während der Katalyse zur Synthese einer DNA-Kette, die komplementär zu einer einzelsträngigen DNA ist, noch verlängert wird, und

   wobei die thermophile DNA-Polymerase von Teil (a) die Aminosäuresequenz von SEQ ID Nr. 1 umfasst und die DNA-Polymerase von Teil (b) die Aminosäuresequenz von SEQ ID Nr. 6 oder SEQ ID Nr. 7 umfasst.

2. Eine DNA-Sequenz, die für die Aminosäuresequenz von SEQ ID Nr. 1, SEQ ID Nr. 6 oder SEQ ID Nr. 7 kodiert.

3. Ein rekombinanter Vektor, der die DNA Sequenz gemäß Anspruch 2 trägt.

4. Der rekombinante Vektor gemäß Anspruch 3, der ein rekombinantes Plasmid pDP 5b17, getragen auf *Escherichia coli* HMS 174 (DE3) /pDP 5b17, hinterlegt beim National Institute of Bioscience and Human Technology, the Agency of Industrial Science and Technology, Japan, unter der Zugangsnummer FERM BP-6189, für SEQ ID Nr. 6 oder ein rekombinantes Plasmid pDP 5c4, getragen auf Escherichia coli HMS 174 (DE3) / pDP 5c4, hinterlegt beim National Institute of Bioscience and Human Technology, the Agency of Industrial Science and Technology, Japan, unter der Zugangsnummer FERM BP-6190, für SEQ ID Nr. 7 ist.

5. Ein Verfahren zur Herstellung einer DNA-Polymerase, umfassend die Kultivierung einer Zelle, transformiert mit einem Expressionsvektor, der die DNA-Sequenz gemäß Anspruch 2 trägt, und die Isolierung und Reinigung des in dem Kulturmedium erzeugten Ziel-Enzyms umfasst.

## Revendications

1. ADN polymérase thermophile, préparée en modifiant artificiellement la séquence d'acides aminés de l'ADN polymérase Pfu de sorte que soit

   (a) l'allongement de la chaîne d'ADN synthétisée peut ne pas être terminé de manière intermédiaire durant la catalyse pour la synthèse de la chaîne d'ADN complémentaire d'un ADN simple brin, ou
   (b) la chaîne d'ADN synthétisée peut être allongée davantage durant la catalyse pour la synthèse de la chaîne d'ADN complémentaire d'un ADN simple brin, et

   où l'ADN polymérase thermophile de la partie (a), comprend la séquence d'acides aminés de la SEQ ID N° 1, et l'ADN polymérise de la partie (b), comprend la séquence d'acides aminés de la SEQ ID N° 6 ou SEQ ID N° 7.

2. Séquence d'ADN codant pour la séquence d'acides aminés de la SEQ ID N°1, SEQ ID N°6 ou SEQ ID N°7.

3. Vecteur recombinant portant la séquence d'ADN selon la revendication 2.

4. Vecteur recombinant selon la revendication 3, qui est un plasmide recombinant pDP 5b17 porté sur une *Escherichia coli* HMS 174 (DE3)/pDP 5b17 déposé au National Intitute of Bioscience and Human Technology, Agency of Industrial science and Technology, Japon sous le numéro d'accès FERM BP-6189 pour la SEQ ID N°6 ou un plasmide recombinant pDP 5c4 porté sur une *Escherichia coli* HMS 174 (DE3)/pDP 5c4 déposé au National Intitute of Bioscience and Human Technology, Agency of Industrial science and Technology, Japon sous le numéro d'accès FERM BP-6190 pour la SEQ ID N°7.

5. Procédé de préparation d'une ADN polymérase, consistant à mettre en culture une cellule transformée avec un vecteur d'expression portant la séquence d'ADN de la revendication 2 et isoler et purifier l'enzyme cible produite dans le milieu de culture.

*Fig. 1*

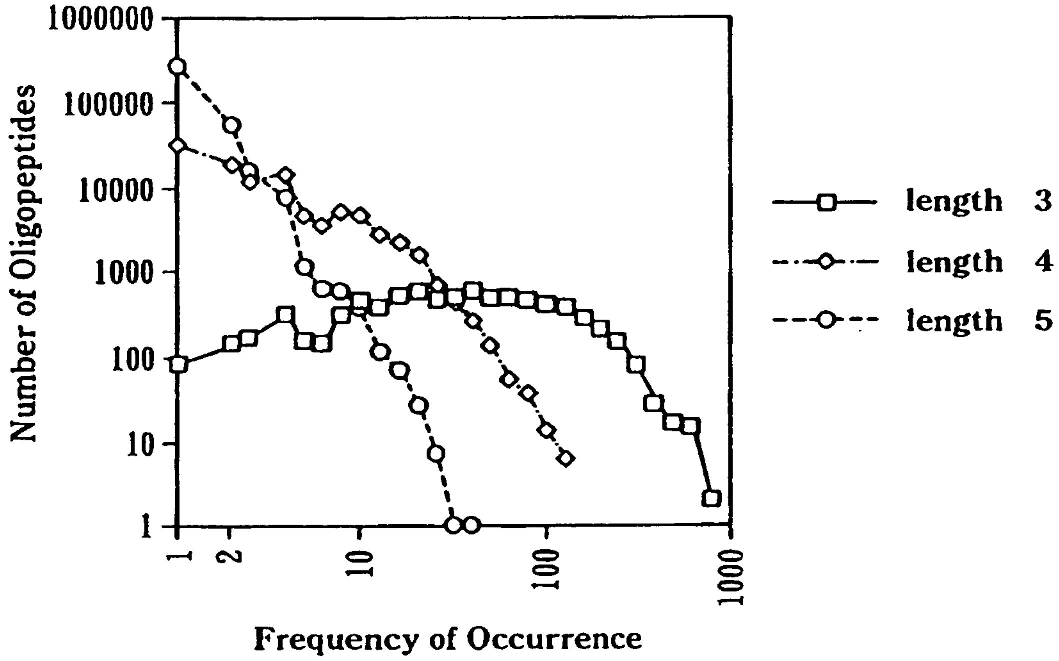
1000000
100000
10000
1000
100
10
1
Number of Oligopeptides
1
2
10
100
1000
Frequency of Occurrence
length 3
length 4
length 5

*Fig. 2*

(1)

1 20
12345678901234567890
LGMSMGKIKIDALIDNTYKT

Sequence (1)

(2)

GMSM ← Aj-oligopeptide (j=5, i=4) of length 4, containing the 5-th Met of the Sequence (1)
MSMG
SMGK
MGKI ← Aj-oligopeptide (j=5, i=1)

| (3) | (4) | (5) | (6) |
|---|---|---|---|
| GMSN | MSNG | SNGK | NGKI |
| GMSD | MSDG | SDGK | DGKI |
| GMSA | MSAG | SAGK | AGKI |
| GMSR | MSRG | SRGK | RGKI |
| GMSI | MSIG | SIGK | IGKI |
| GMSG | MSGG | SGGK | GGKI |
| GMSQ | MSQG | SQGK | QGKI |
| GMSE | MSEG | SEGK | EGKI |
| GMSC | MSCG | SCGK | CGKI |
| GMSS | MSSG | SSGK | SGKI |
| GMSY | MSYG | SYGK | YGKI |
| GMSW | MSWG | SWGK | WGKI |
| GMST | MSTG | STGK | TGKI |
| GMSV | MSVG | SVGK | VGKI |
| GMSH | MSHG | SHGK | HGKI |
| GMSF | MSFG | SFGK | FGKI |
| GMSP | MSPG | SPGK | PGKI |
| GMSM | MSMG | SMGK | MGKI |
| GMSK | MSKG | SKGK | KGKI |
| GMSL | MSLG | SLGK | LGKI |

Xi-oligopeptide (i=4) for the oligopeptide GMSM of length 4, containing the 5-th Met of the Sequence (1)

Xi-oligopeptide (i=1) for the oligopeptide MGKI of length 4, containing the 5-th Met of the Sequence (1)

*Fig. 3*

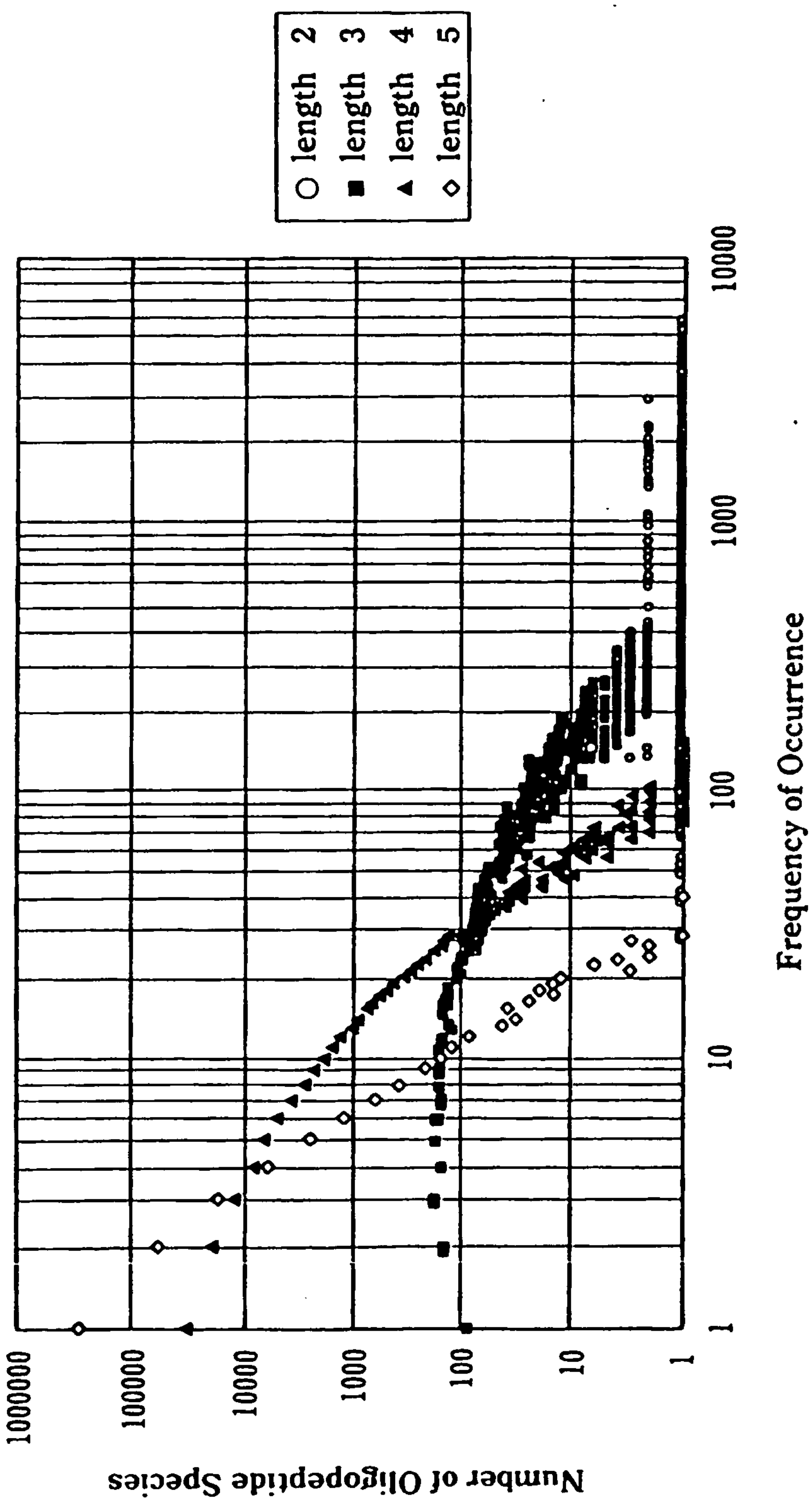
length 2
length 3
length 4
length 5
Frequency of Occurrence
Number of Oligopeptide Species
1
10
100
1000
10000
1
10
100
1000
10000
100000
1000000

*Fig. 4*

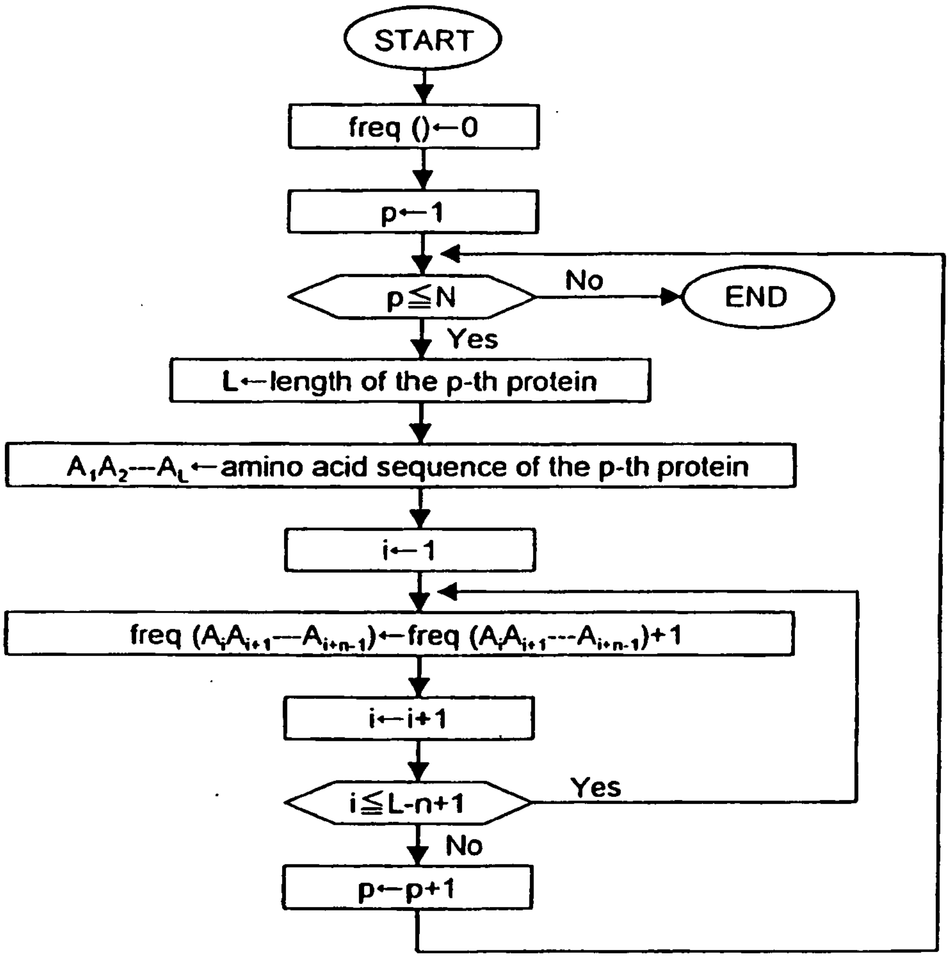
START
freq ()←0
p←1
p≦N
No
END
Yes
L←length of the p-th protein
A1A2---AL←amino acid sequence of the p-th protein
i←1
freq (AiAi+1---Ai+n-1)←freq (AiAi+1---Ai+n-1)+1
i←i+1
i≦L-n+1
Yes
No
p←p+1

Fig. 5

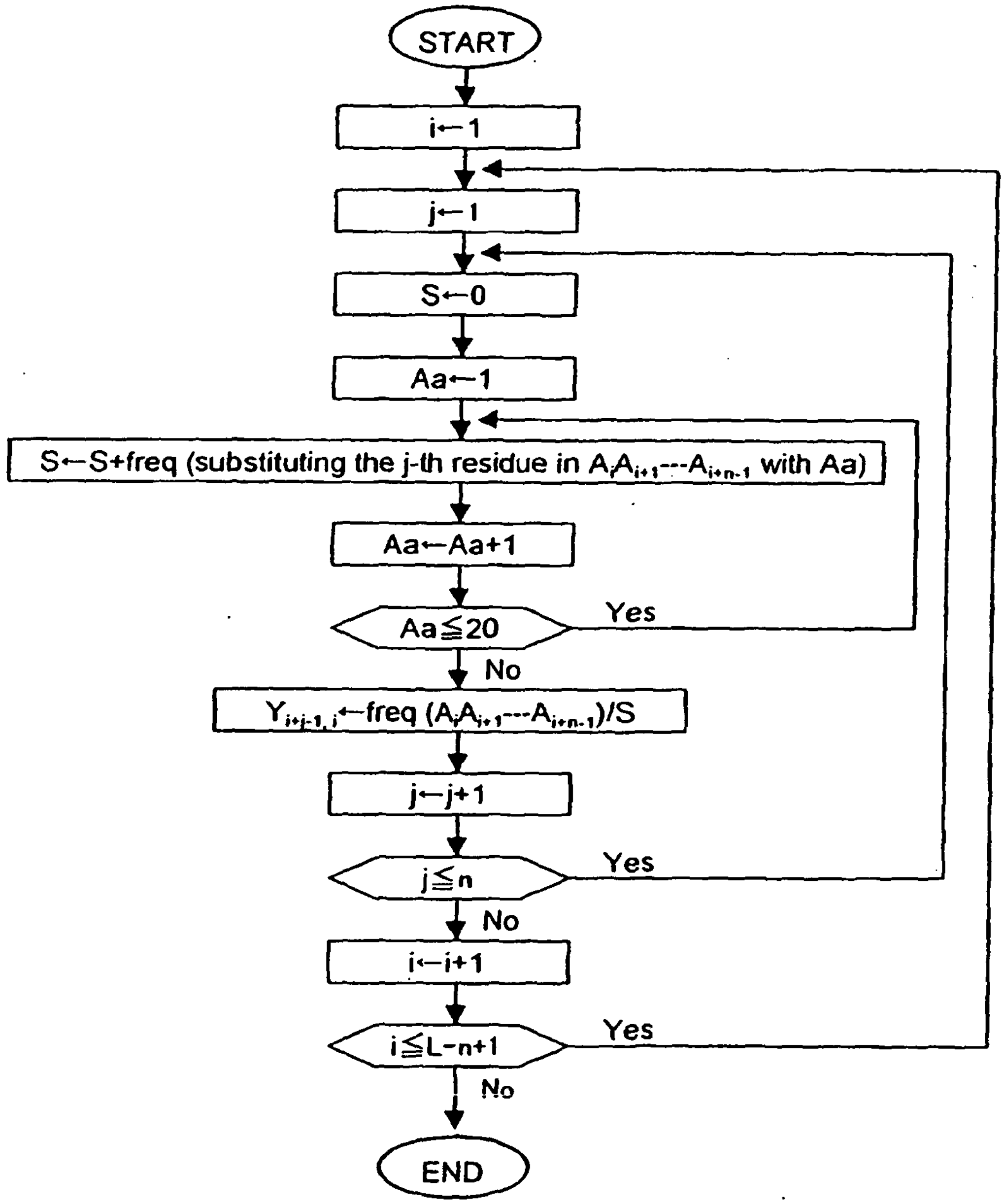
START
i←1
j←1
S←0
Aa←1
S←S+freq (substituting the j-th residue in A_iA_{i+1}---A_{i+n-1} with Aa)
Aa←Aa+1
Aa≦20
Yes
No
Y_{i+j-1, i}←freq (A_iA_{i+1}---A_{i+n-1})/S
j←j+1
j≦n
Yes
No
i←i+1
i≦L-n+1
Yes
No
END

*Fig. 6*

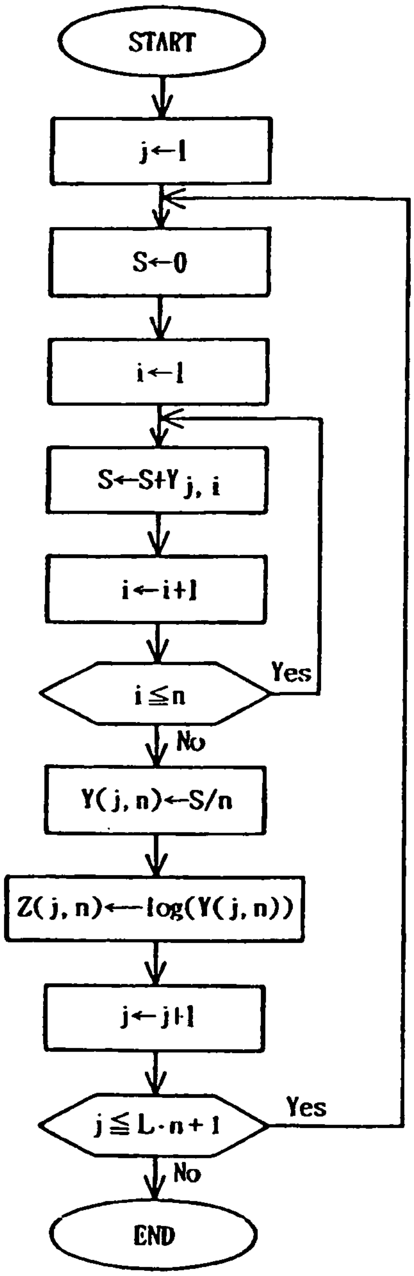
START
j←1
S←0
i←1
S←S+Y j, i
i←i+1
i≦n
Yes
No
Y(j, n)←S/n
Z(j, n)←−log(Y(j, n))
j←j+1
j≦L-n+1
Yes
No
END

*Fig. 7*

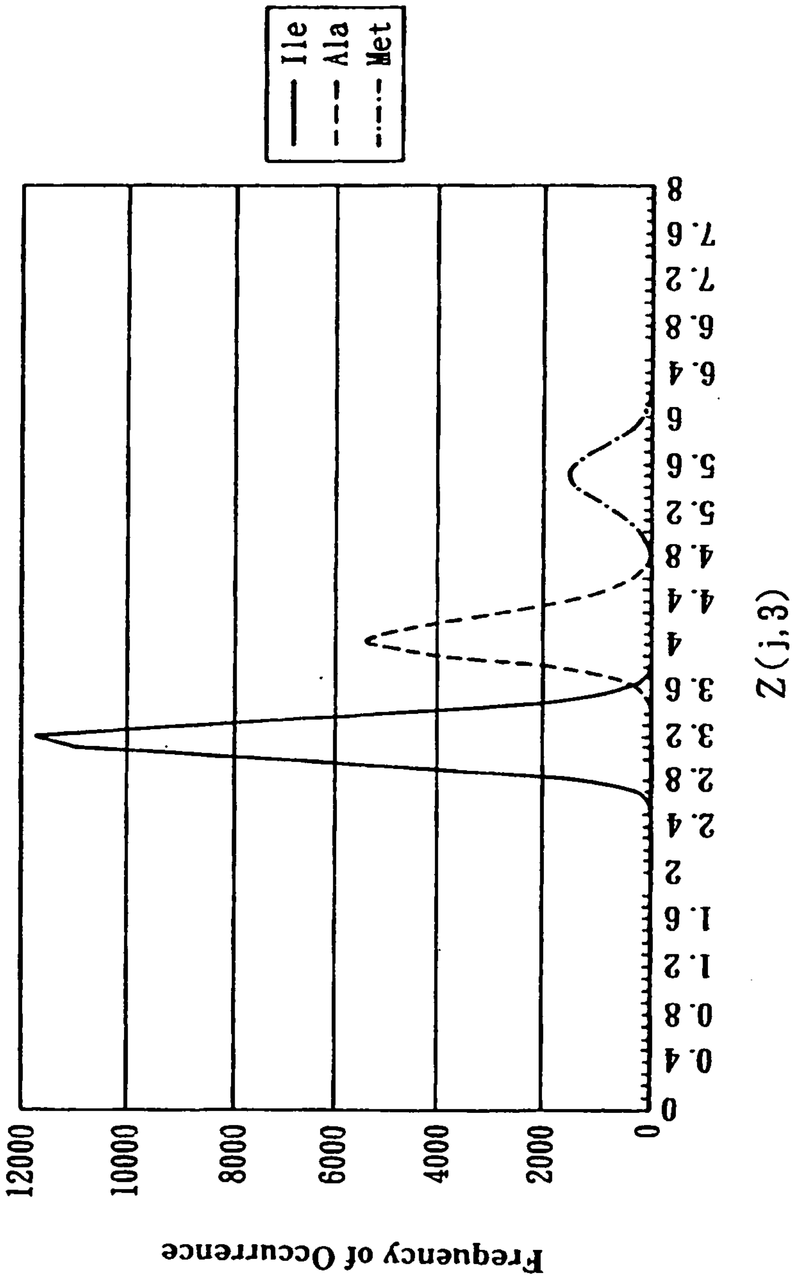
Ile
Ala
Met
Frequency of Occurrence
Z(j,3)
0
2000
4000
6000
8000
10000
12000
0.4
0.8
1.2
1.6
2
2.4
2.8
3.2
3.6
4
4.4
4.8
5.2
5.6
6
6.4
6.8
7.2
7.6
8

*Fig. 8*

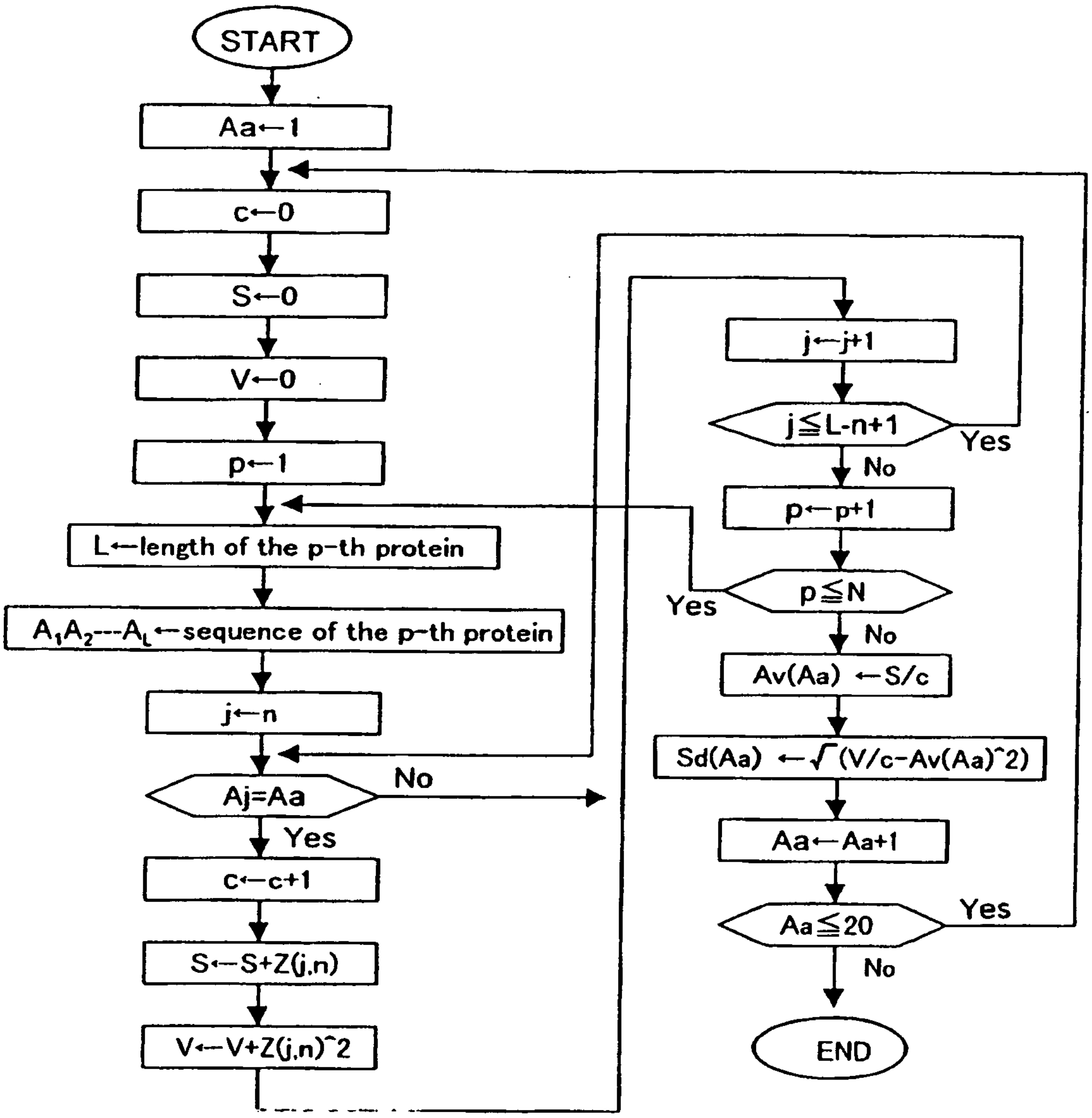
START
Aa←1
c←0
S←0
V←0
p←1
L←length of the p-th protein
A1A2---AL←sequence of the p-th protein
j←n
Aj=Aa
No
Yes
c←c+1
S←S+Z(j,n)
V←V+Z(j,n)^2
j←j+1
j≦L-n+1
Yes
No
p←p+1
p≦N
Yes
No
Av(Aa) ←S/c
Sd(Aa) ←√(V/c-Av(Aa)^2)
Aa←Aa+1
Aa≦20
Yes
No
END

*Fig. 9*

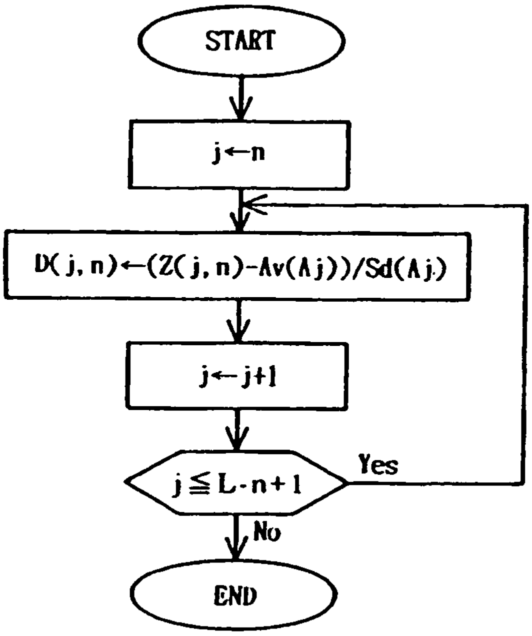

*Fig. 10*

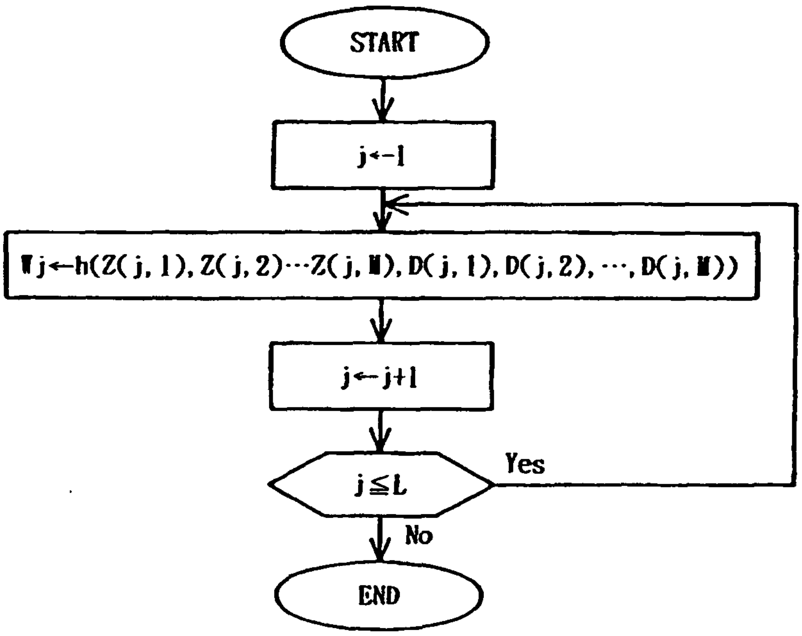

Fig. 11

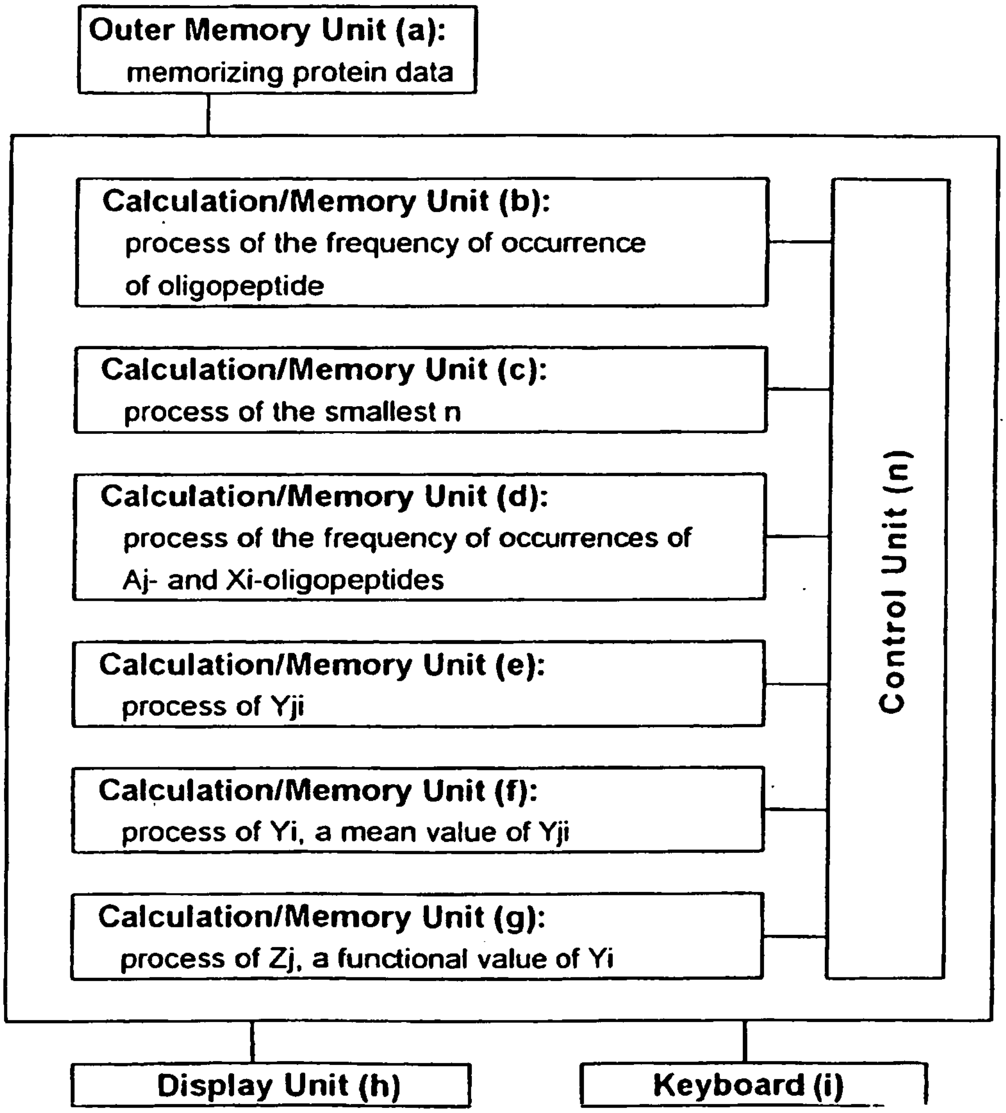
Outer Memory Unit (a):
memorizing protein data
Calculation/Memory Unit (b):
process of the frequency of occurrence of oligopeptide
Calculation/Memory Unit (c):
process of the smallest n
Calculation/Memory Unit (d):
process of the frequency of occurrences of Aj- and Xi-oligopeptides
Calculation/Memory Unit (e):
process of Yji
Calculation/Memory Unit (f):
process of Yi, a mean value of Yji
Calculation/Memory Unit (g):
process of Zj, a functional value of Yi
Control Unit (n)
Display Unit (h)
Keyboard (i)

Fig. 12

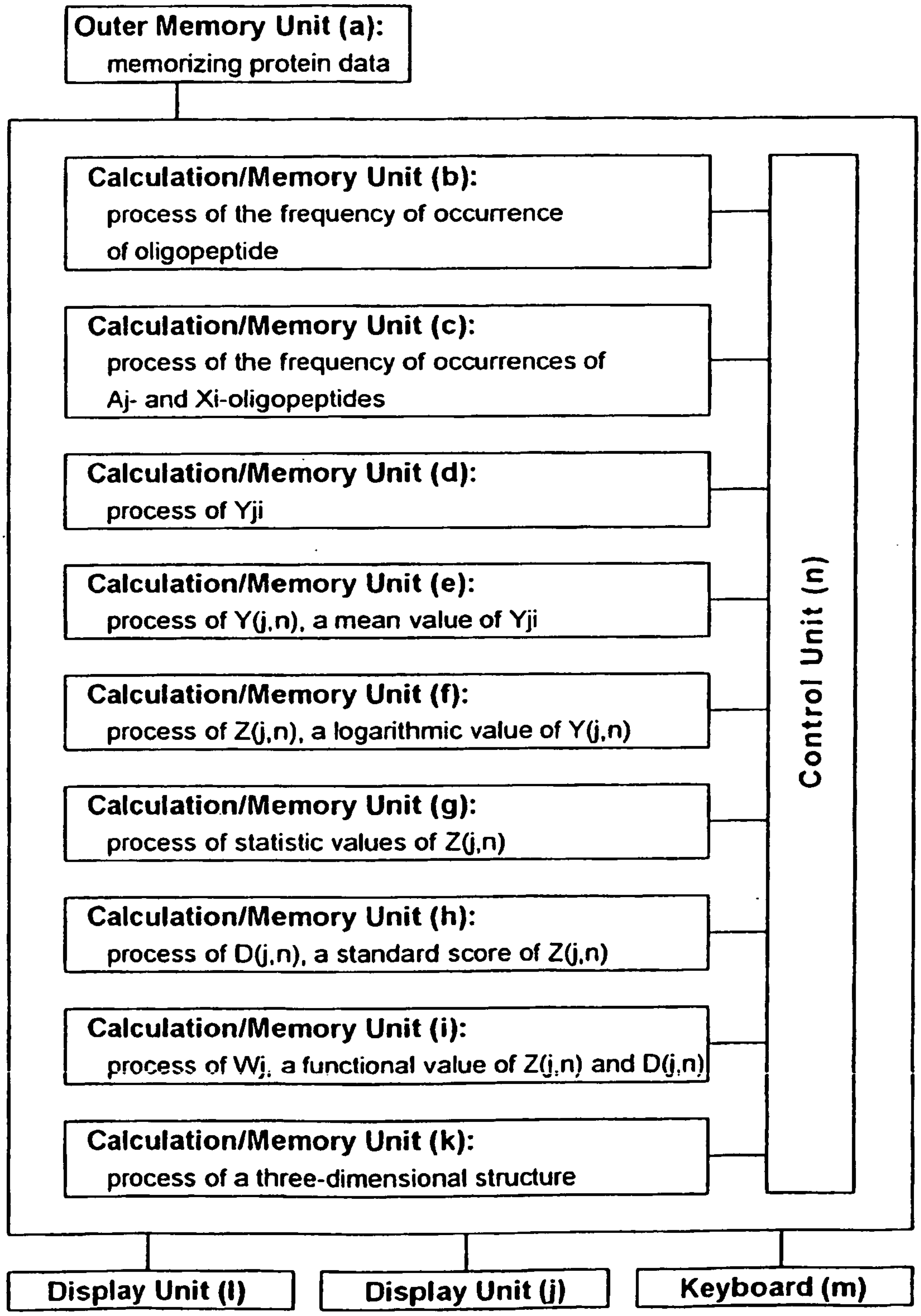
Outer Memory Unit (a):
memorizing protein data
Calculation/Memory Unit (b):
process of the frequency of occurrence of oligopeptide
Calculation/Memory Unit (c):
process of the frequency of occurrences of Aj- and Xi-oligopeptides
Calculation/Memory Unit (d):
process of Yji
Calculation/Memory Unit (e):
process of Y(j,n), a mean value of Yji
Calculation/Memory Unit (f):
process of Z(j,n), a logarithmic value of Y(j,n)
Calculation/Memory Unit (g):
process of statistic values of Z(j,n)
Calculation/Memory Unit (h):
process of D(j,n), a standard score of Z(j,n)
Calculation/Memory Unit (i):
process of Wj, a functional value of Z(j,n) and D(j,n)
Calculation/Memory Unit (k):
process of a three-dimensional structure
Control Unit (n)
Display Unit (l)
Display Unit (j)
Keyboard (m)

Fig. 13

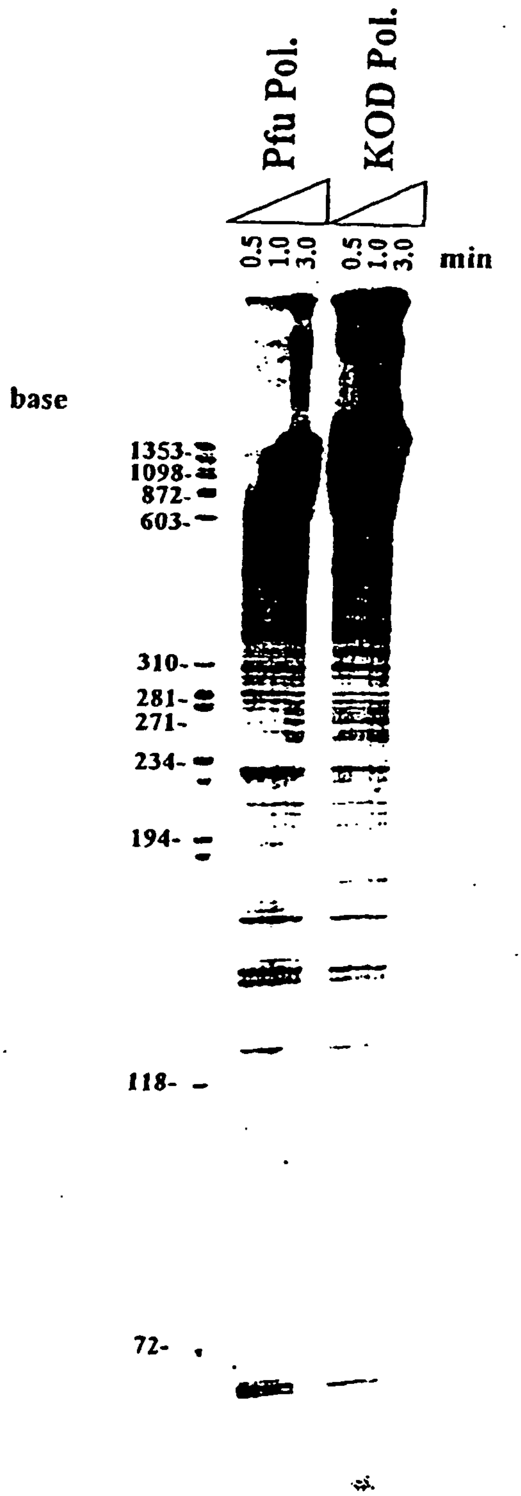
Pfu Pol.
KOD Pol.
0.5
1.0
3.0
0.5
1.0
3.0
min
base
1353-
1098-
872-
603-
310-
281-
271-
234-
194-
118-
72-

*Fig. 14*

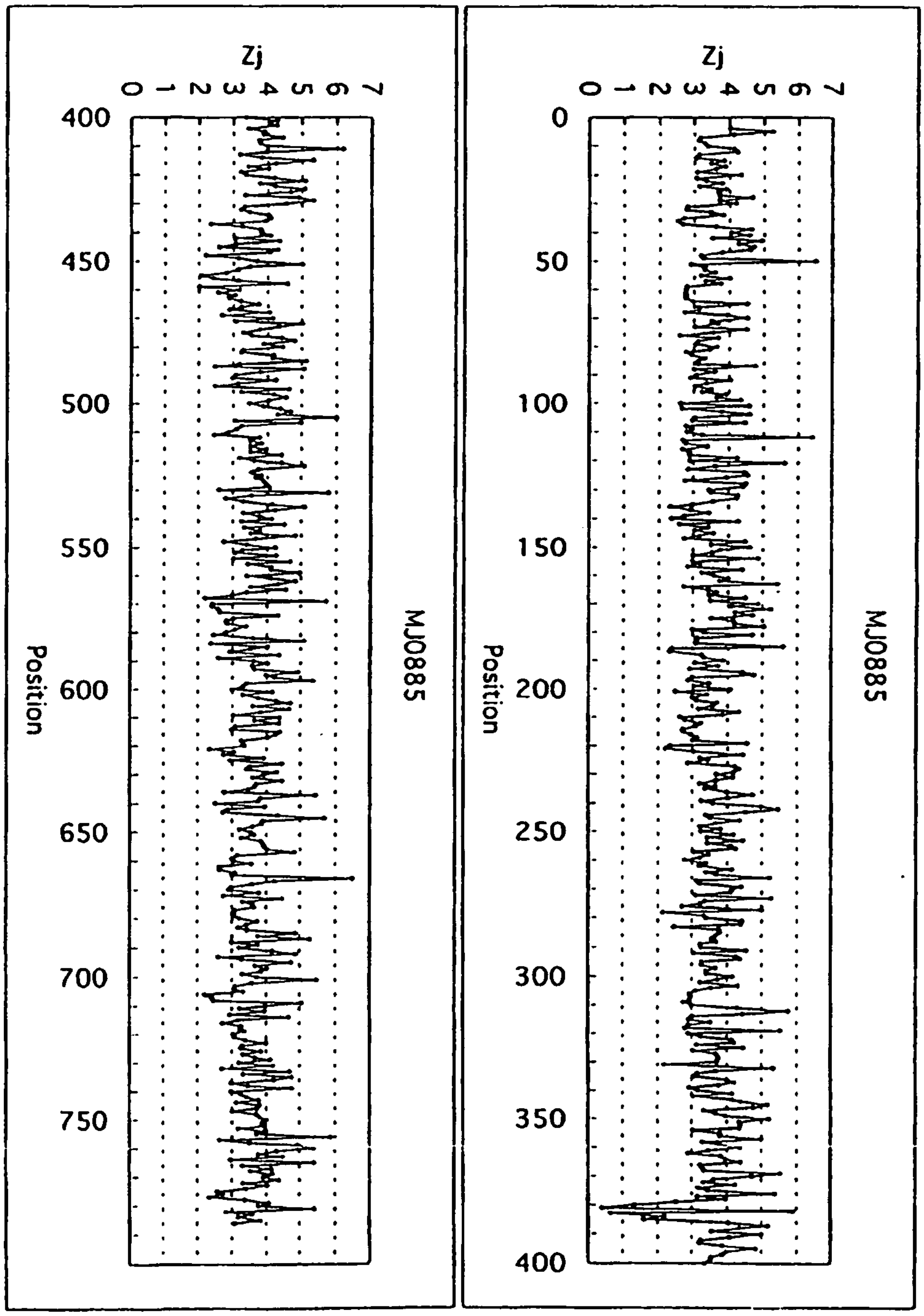

*Fig. 15*

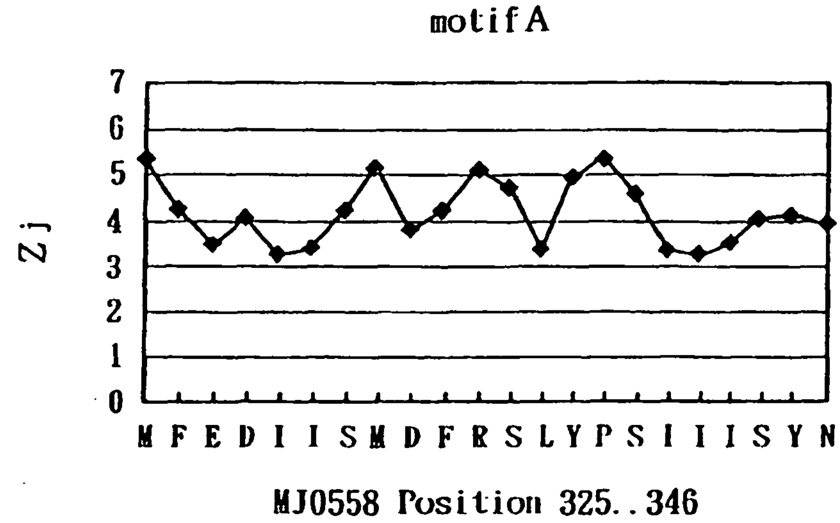
motifA
Zj
7
6
5
4
3
2
1
0
M F E D I I S M D F R S L Y P S I I I S Y N
MJ0558 Position 325..346

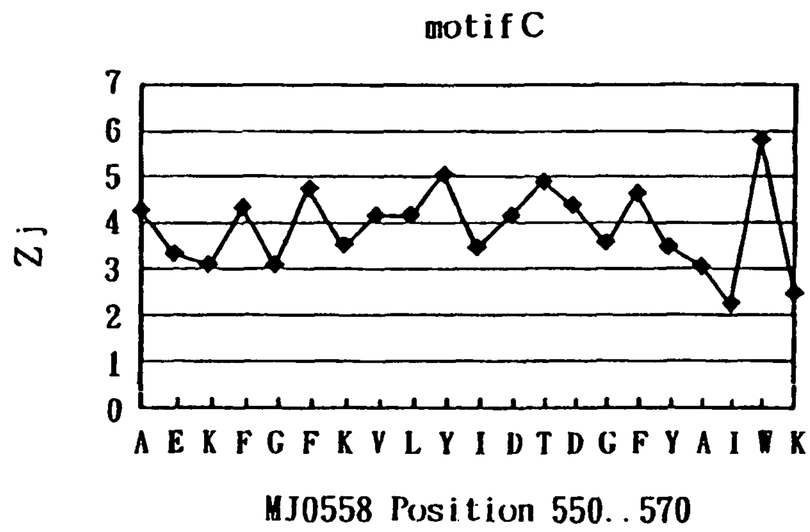
motifC
Zj
7
6
5
4
3
2
1
0
A E K F G F K V L Y I D T D G F Y A I W K
MJ0558 Position 550..570

*Fig. 16*

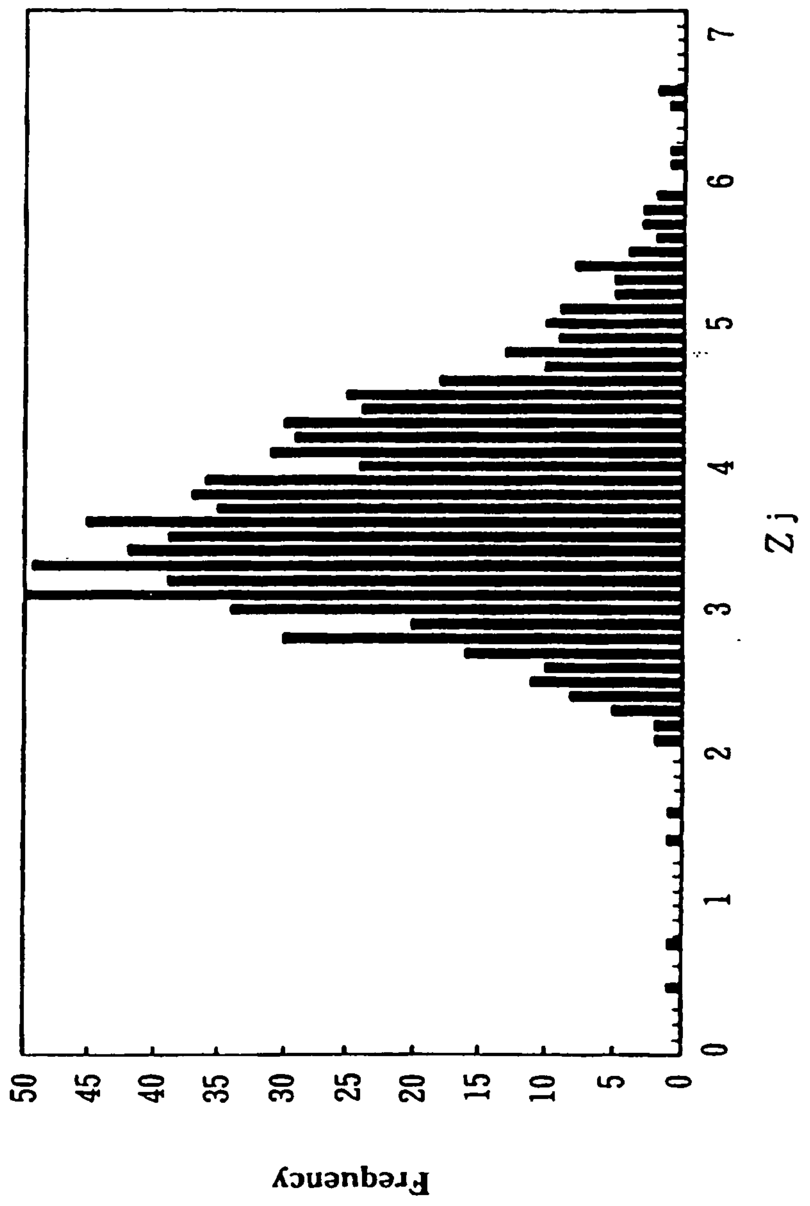

*Fig. 17*

motif C

```
    550                 570
MJ  AEKFGFKVLYIDTDGFYAIWK
KOD EEKYGFKVIYSDTDGFFATIP
Pfu EEKFGFKVLYIDTDGLYATIP
```

*Fig. 18*

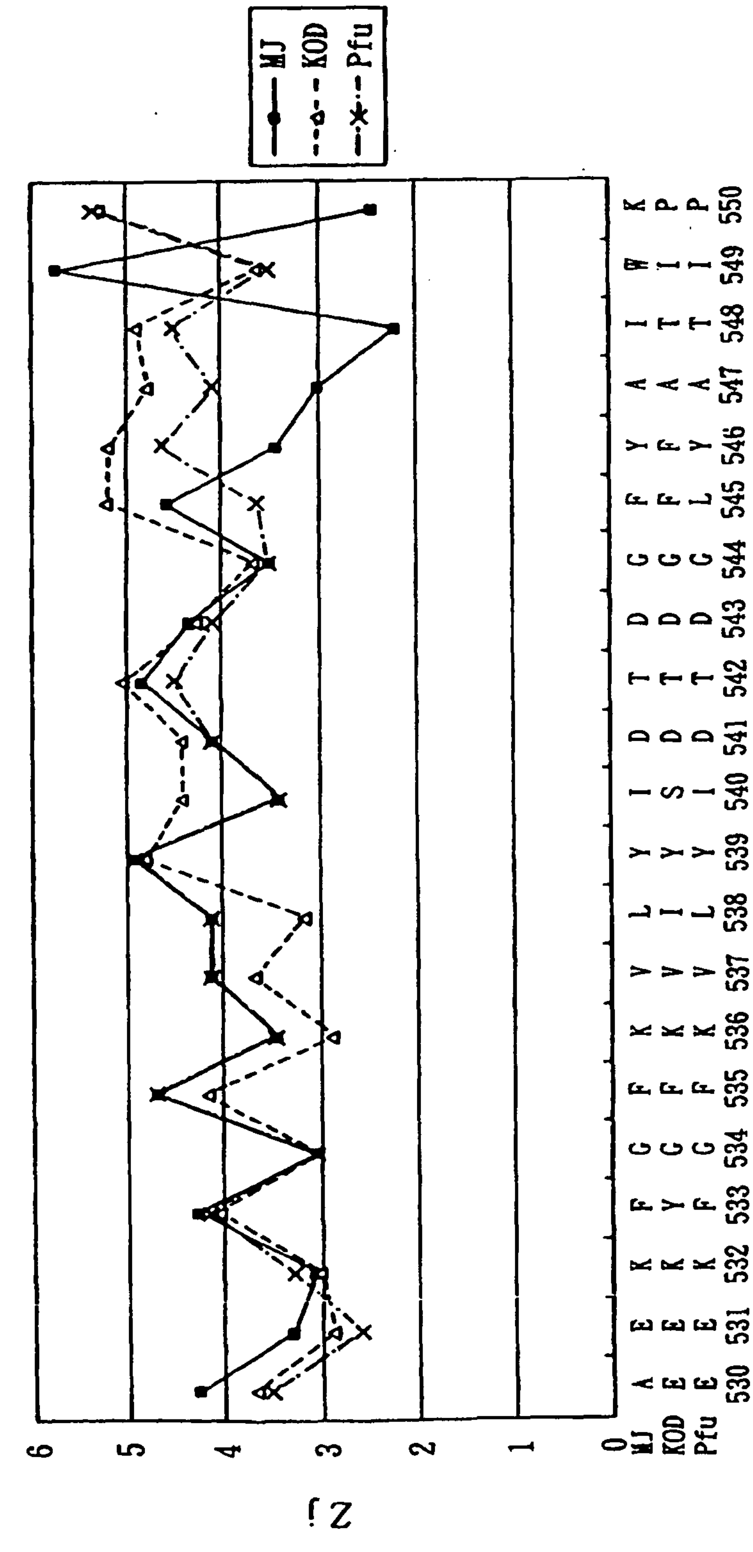
MJ
KOD
Pfu
Zj
0
1
2
3
4
5
6
MJ A E K F G F K V L Y I D T D G F Y A I W K
KOD E E K Y G F K V I Y S D T D G F F A T I P
Pfu E E K F G F K V L Y I D T D C L Y A T I P
530 531 532 533 534 535 536 537 538 539 540 541 542 543 544 545 546 547 548 549 550

*Fig. 19*

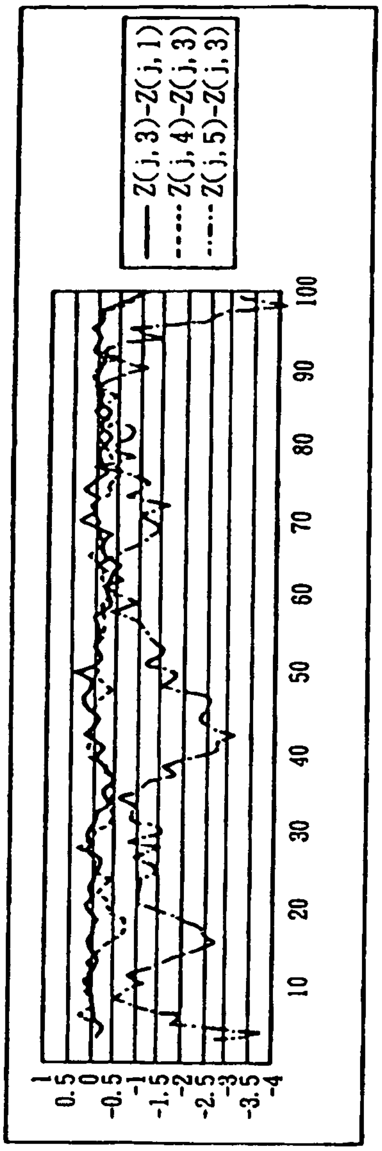
Z(j,3)-Z(j,1)
Z(j,4)-Z(j,3)
Z(j,5)-Z(j,3)
1
0.5
0
-0.5
-1
-1.5
-2
-2.5
-3
-3.5
-4
10
20
30
40
50
60
70
80
90
100

*Fig. 20*

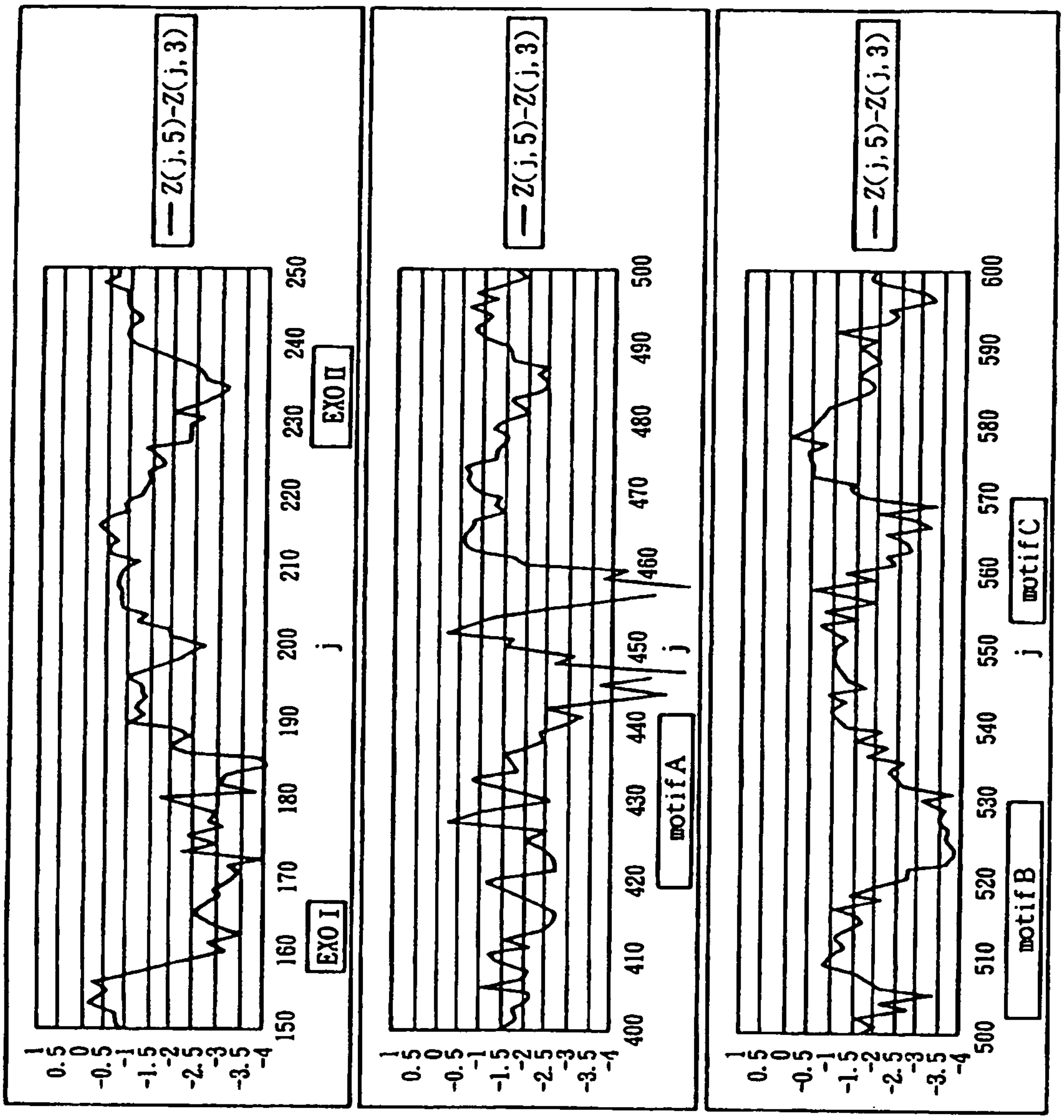

*Fig. 21*

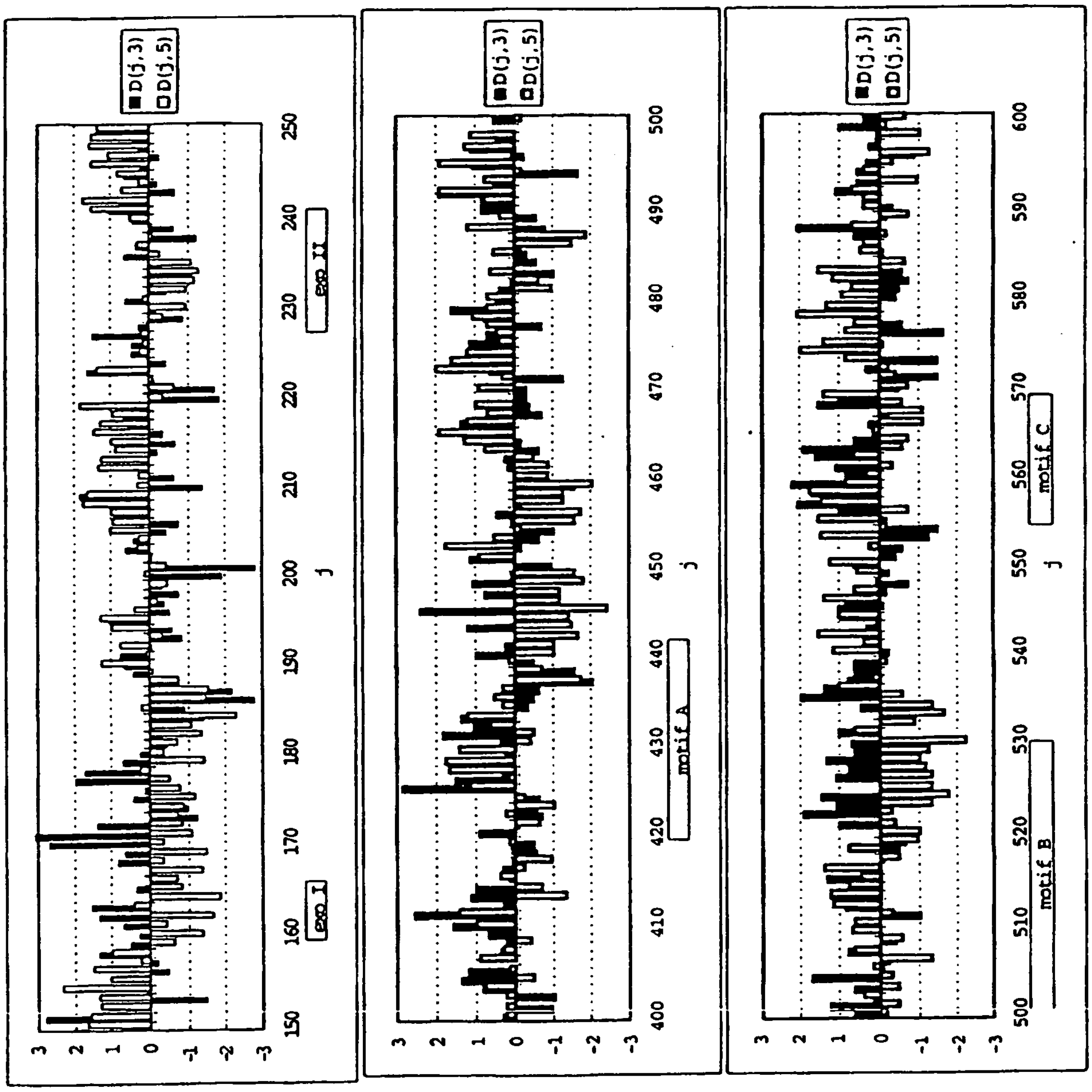

*Fig. 22*

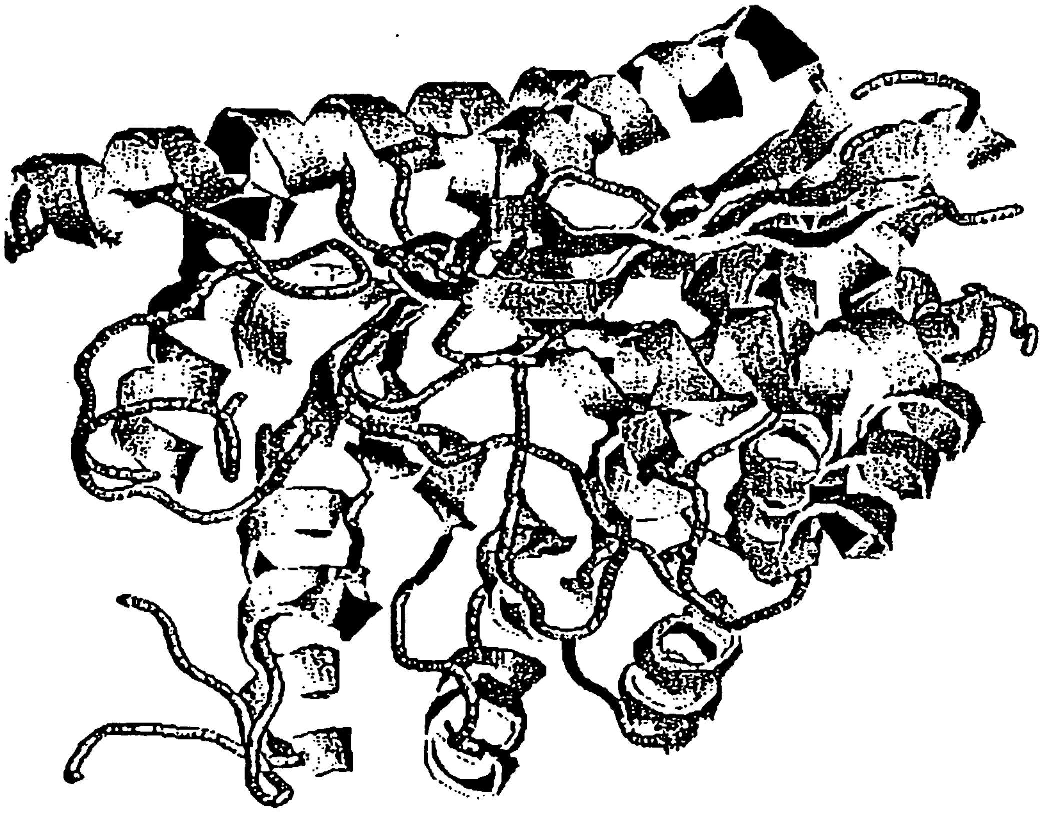

*Fig. 23*

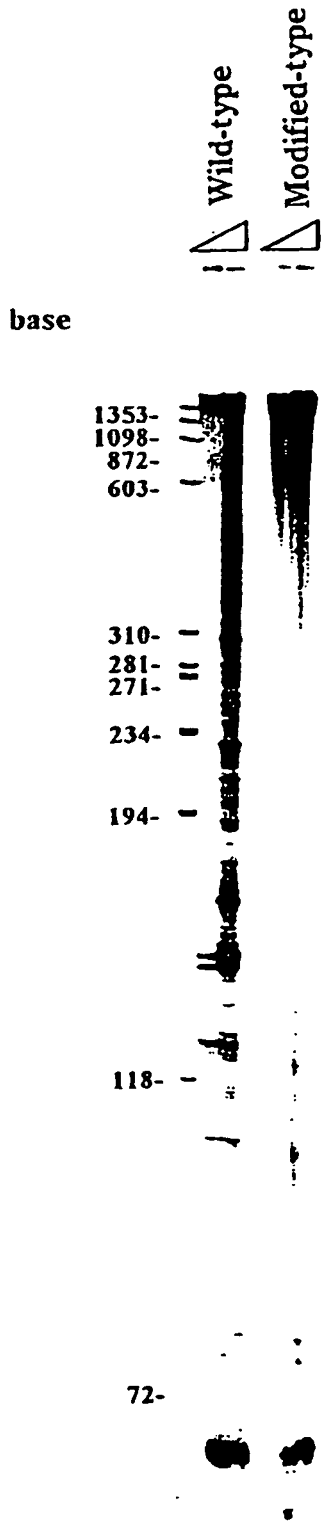
Wild-type
Modified-type
base
1353-
1098-
872-
603-
310-
281-
271-
234-
194-
118-
72-

Fig. 24

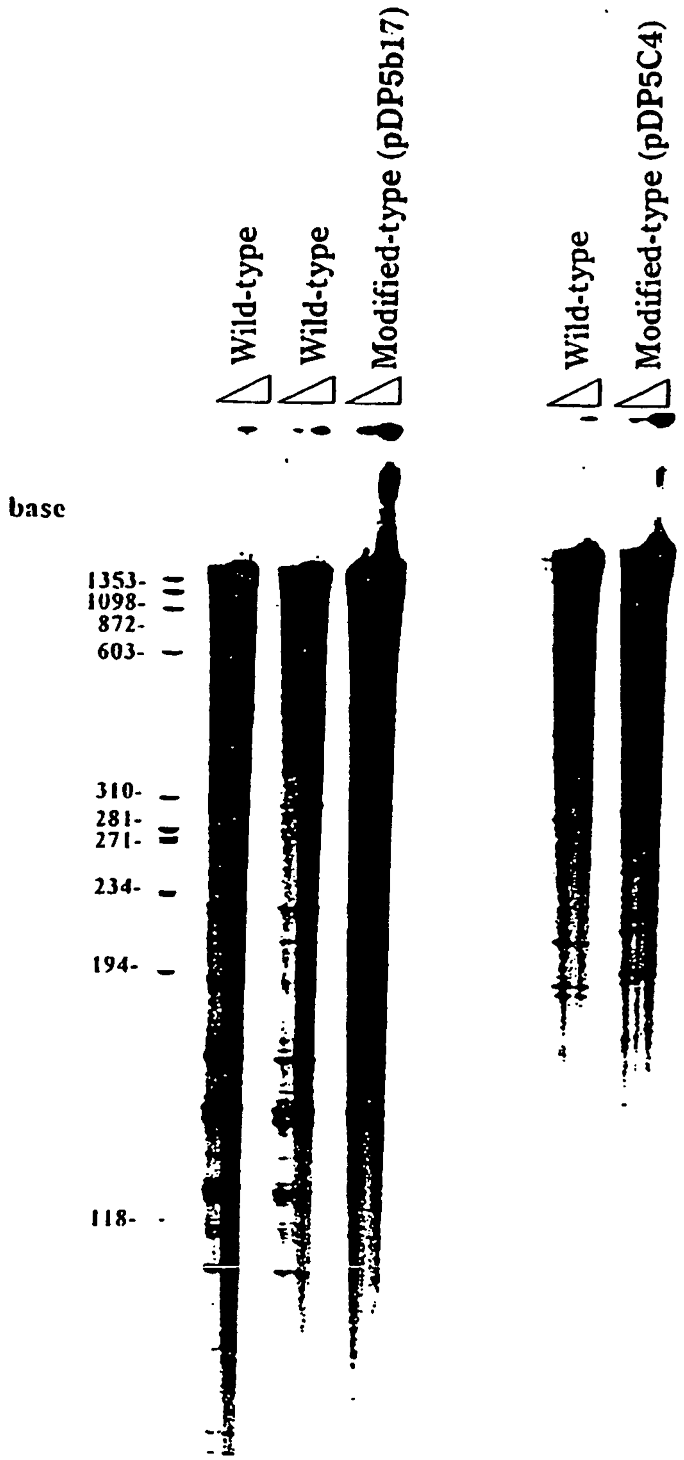
Wild-type
Wild-type
Modified-type (pDP5b17)
Wild-type
Modified-type (pDP5C4)
base
1353-
1098-
872-
603-
310-
281-
271-
234-
194-
118-

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Fraser et al.** *Science,* 1995, vol. 270, 397-403
- **Fleischman et al.** *Science,* 1995, vol. 269, 496-512
- **Bult et al.** *Science,* 1996, vol. 273, 1058-1073
- **Bult et al.** *Science,* 1996, vol. 273, 1053-1073
- **Peitsch.** *Proceedings of the Fifth International Conference on Intelligent Systems for Molecular Biology,* 1997, vol. 5, 234-236
- *Strategies,* 1996, vol. 9, 3-4
- *Nucleic Acids Research,* 1993, vol. 21, 259-265